# EUROPEAN PATENT SPECIFICATION

(11) **EP 2 148 667 B1**
(45) Date of publication and mention of the grant of the patent: **22.05.2013**
(21) Application number: 08733752.3
(22) Date of filing: 11.04.2008
(51) Int. Cl.: A61K 31/047, A61P 27/02, A61P 27/06

(54) **USE OF CYCLOHEXANEHEXOL DERIVATIVES IN THE TREATMENT OF OCULAR DISEASES**
VERWENDUNG VON CYCLOHEXANHEXOL-DERIVATEN BEI DER BEHANDLUNG VON AUGENERKRANKUNGEN
UTILISATION DE DÉRIVÉS DE CYCLOHEXANEHEXOL DANS LE TRAITEMENT DE MALADIES OCULAIRES

(30) Priority: 12.04.2007 US 922998 P
(43) Date of publication of application: 03.02.2010
(73) Proprietor: Waratah Pharmaceuticals, Inc., Toronto, ON M5G 1L7 (CA)
(72) Inventor: CRUZ, Antonio, Toronto, Ontario M5P 2T5 (CA)
(74) Representative: Holliday, Louise Caroline
(86) International application number: PCT/CA2008/000703
(87) International publication number: WO 2008/124940

(56) References cited:
- WO-A1-91/09601
- CA-A1- 2 105 530
- US-A- 4 201 706
- US-A1- 2006 148 905
- US-A1- 2006 189 582
- US-A1- 2007 078 099

## Description

### FIELD OF THE INVENTION

The invention relates to compositions and treatments for macular degeneration

### BACKGROUND OF THE INVENTION

Extracellular deposits consisting of misfolded and aggregated proteins are present in aging human eyes and in eyes afflicted by ocular diseases and disorders such as age-related macular degeneration (AMD). Drusen deposits, which are associated with aging and age-related macular degeneration, are found beneath the basement membrane of the retinal pigmented epithelium and the inner collagenous layer of the Bruch membrane. Drusen contains a variety of lipids and proteins that are commonly shared with amyloid deposits including vitronectin, amyloid P, apolipoprotein E, and amyloid β (Luibl, V et al, J Clin Invest. 116:378- 385, 2006). Primary open angle glaucoma (POAG) and pseudo-exfoliation syndrome (PEX) are also characterized by formation of amyloid-like material. In POAG destruction of neuronal tissue with amyloid deposit formation is found in the atrophic optic nerve, and in PEX deposits are detectable on the walls of the anterior chamber, in particular on the anterior lens capsule and in the chamber angle. Amyloid β and serine proteinase inhibitors have also been found in aqueous humour from cataractous eyes (Janciauskiene and Krakau, Documenta Ophthalmologica 106: 215-223, 2003). Intravitreal injection of Aβ₁₋₄₂ was found to cause apoptosis of inter-neurones in the photoreceptor and inner nuclear layer of the retina at 48 hours, a significant reduction in the ganglion cell layer (Jen, LS et al, Nature 1998: 392: 140-1; Walsh DT, et al, Neurobiol Dis 2002: 10:20-7), a reduction in the retinal surface area, and a marked activation of Muller glial cells and microglia.

### SUMMARY OF THE INVENTION

The present invention relates to methods for treating an ocular disease in a subject comprising administering a therapeutically effective amount of an isolated and pure cyclohexanehexol compound, in particular a scyllo-cyclohexanehexol compound or analog or derivative thereof. The methods of the invention can be used therapeutically or can be used prophylactically in a subject susceptible to ocular diseases.

The invention also provides a method for treating an ocular disease in a subject comprising administering to the subject a therapeutically effective amount of one or more cyclohexanehexol compound, or a pharmaceuti catty acceptable salt thereof, or a medicament comprising a cyclohexanehexol compound and a pharmaceutically acceptable carrier, excipient, or vehicle, which results in beneficial effects following treatment. In particular, the invention relates to a method for the treatment of a subject suffering from an ocular disease comprising administering at least one cyclohexanehexol compound or a pharmaceutically acceptable salt thereof, to the subject in an amount effective to treat the subject.

In an aspect, the invention relates to a method of treatment comprising administering a therapeutically effective amount of one or more cyclohexanehexol compound, a pharmaceutically acceptable salt thereof, or a medicament comprising a cyclohexanehexol compound, and a pharmaceutically acceptable carrier, excipient, or vehicle, which upon administration to a subject with symptoms of an ocular disease produces beneficial effects, in particular sustained beneficial effects.

In particular aspects, beneficial effects are evidenced by one or more of the following: modulation (e.g., inhibition, reversal, or reduction) of assembly, folding, accumulation, rate of aggregation and/or clearance of amyloid β or oligomers or aggregates comprising amyloid β, in particular prevention, reduction or inhibition of amyloid β aggregation or assembly of oligomers or aggregates comprising amyloid β (e.g., drusen) in ocular cells, reversal or reduction of amyloid β or oligomers or aggregates comprising amyloid β after the onset of symptoms of an ocular disease, dissolution and/or disruption of amyloid β, or oligomers or aggregates comprising amyloid β, enhanced clearance of amyloid β, or oligomers or aggregates comprising amyloid β, reduction or inhibition of vascular endothelial growth factor (VEGF) or VEGF activity, and, slowing or arrest of the progress of an ocular disease.

In an aspect, a method is provided for treating a mammal in need of improved ocular function, wherein the mammal has no diagnosed disease, disorder, infirmity or ailment known to impair or otherwise diminish ocular function, comprising the step of administering to the mammal a therapeutically effective amount for improving ocular function of a cyclohexanehexol compound, a pharmaceutically acceptable salt thereof, or a dietary supplement comprising a cyclohexanehexol compound, or a nutraceutically acceptable derivative thereof.

In a further aspect, the invention provides a method involving administering to a subject a therapeutically effective amount of a cyclohexanehexol compound, a pharmaceutically acceptable salt thereof, or a medicament comprising a cyclohexanehexol compound and a pharmaceutically acceptable carrier, excipient, or vehicle which modulates folding, oligomerization and/or aggregation of amyloid in ocular cells.

In a further aspect, the invention provides a method involving administering to a subject a therapeutically effective amount of a cyclohexanehexol compound, a pharmaceutically acceptable salt thereof, or a medicament comprising a cyclohexanehexol compound and a pharmaceutically acceptable carrier, excipient, or vehicle which causes dissolution/disruption of pre-existing amyloid, amyloid oligomers or aggregates in ocular cells or tissues.

In an aspect, the invention provides a method for preventing or inhibiting assembly or slowing deposition of amyloid in ocular cells comprising administering a therapeutically effective amount for preventing or inhibiting assembly or slowing deposition of amyloid or oligomers or aggregates comprising amyloid in ocular cells of a cyclohexanehexol compound, a pharmaceutically acceptable salt thereof, or a medicament comprising a cyclohexanehexol compound and a pharmaceutically acceptable carrier, excipient, or vehicle.

In an embodiment, the invention provides a method of reversing or reducing amyloid or oligomers and/or aggregates comprising amyloid in ocular cells after the onset of symptoms of an ocular disease in a subject comprising administering to the subject a therapeutically effective amount of a cyclohexanehexol compound, a pharmaceutically acceptable salt thereof, or a medicament comprising a cyclohexanehexol compound and a pharmaceutically acceptable carrier, excipient, or vehicle.

In an aspect, the invention provides a method for enhancing clearance of amyloid or oligomers or aggregates comprising amyloid in ocular cells in a subject comprising administering a therapeutically effective amount for enhancing clearance of amyloid or oligomers or aggregates comprising amyloid in ocular cells, of a cyclohexanehexol compound, a pharmaceutically acceptable salt thereof, or a medicament comprising a cyclohexanehexol compound and a pharmaceutically acceptable carrier, excipient, or vehicle.

In an aspect, the invention provides a method for amelioriating symptoms or onset of an ocular disease comprising administering a therapeutically effective amount for amelioriating symptoms or onset of an ocular disease of a cyclohexanehexol compound, a pharmaceutically acceptable salt thereof, or a medicament comprising a cyclohexanehexol compound and a pharmaceutically acceptable carrier, excipient, or vehicle.

In an aspect, the invention provides a method for amelioriating progression of an ocular disease comprising administering a therapeutically effective amount for amelioriating progression of the ocular disease of a cyclohexanehexol compound, a pharmaceutically acceptable salt thereof, or a medicament comprising a cyclohexanehexol compound and a pharmaceutically acceptable carrier, excipient, or vehicle.

In an aspect, the invention provides a method for amelioriating progression of AMD, or progression of dry AMD to wet AMD, comprising administering a therapeutically effective amount of a cyclohexanehexol compound, a pharmaceutically acceptable salt thereof, or a medicament comprising a cyclohexanehexol compound and a pharmaceutically acceptable carrier, excipient, or vehicle.

In an aspect, the invention relates to a method of delaying the onset or progression of an ocular disease comprising administering a therapeutically effective amount for delaying the onset or progression of the ocular disease of a cyclohexanehexol compound, a pharmaceutically acceptable salt thereof, or a medicament comprising a cyclohexanehexol compound and a pharmaceutically acceptable carrier, excipient, or vehicle.

In an aspect, the invention relates to a method of delaying the onset or progression of AMD or onset or progression of dry AMD to wet AMD, comprising administering a therapeutically effective amount of a cyclohexanehexol compound, a pharmaceutically acceptable salt thereof, or a medicament comprising a cyclohexanehexol compound and a pharmaceutically acceptable carrier, excipient, or vehicle.

In an aspect, the invention relates to a method of preventing an ocular disease in a subject comprising administering a prophylactically effective amount of a cyclohexanehexol compound, a pharmaceutically acceptable salt thereof, or a medicament comprising a prophylactically effective amount of a cyclohexanehexol compound and a pharmaceutically acceptable carrier, excipient, or vehicle.

In an aspect, the invention provides a method for protecting ocular cells in a subject having an ocular disease comprising administering a prophylactically effective amount of a cyclohexanehexol compound, a pharmaceutically acceptable salt thereof, or a medicament comprising a prophylactically effective amount of a cyclohexanehexol compound and a pharmaceutically acceptable carrier, excipient, or vehicle.

In an aspect, the invention provides a method for administering a cyclohexanehexol compound or a medicament comprising a cyclohexanehexol compound and a pharmaceutically acceptable carrier, excipient, or vehicle in a therapeutically effective amount to patients who need ocular disease treatments while minimizing the occurrence of adverse effects.

In an aspect, the invention provides medicaments for prevention and/or treatment of ocular diseases. Thus, the invention provides a medicament comprising a cyclohexanehexol compound, in particular a therapeutically effective amount of a cyclohexanehexol compound, for treating ocular diseases. More particularly, the invention provides a medicament in a form adapted for administration to a subject to provide beneficial effects to treat ocular diseases. In an aspect, a medicament is in a form such that administration to a subject suffering from an ocular disease results in modulation of of assembly, folding, accumulation, rate of aggregation and/or clearance of amyloid β or oligomers or aggregates comprising amyloid β, in particular prevention, reduction or inhibition of amyloid β aggregation or assembly of oligomers or aggregates comprising amyloid β (e.g., drusen) in ocular cells, reversal or reduction of amyloid β or oligomers or aggregates comprising amyloid β after the onset of symptoms of an ocular disease, dissolution and/or disruption of amyloid β, or oligomers or aggregates comprising amyloid β, enhanced clearance of amyloid β, or oligomers or aggregates comprising amyloid β, reduction or inhibition of VEGF or VEGF activity, and, slowing or arrest of the progress of an ocular disease.

The invention features a medicament comprising a cyclohexanehexol compound in a therapeutically effective amount for modulating amyloid oligomerization and/or aggregation in ocular cells. In an aspect, the invention provides a medicament comprising a cyclohexanehexol compound in a therapeutically effective amount for reducing and/or inhibiting amyloid oligomerization and/or aggregation in ocular cells or dissolving and/or disrupting pre-existing amyloid oligomers or aggregates in ocular cells. The medicament can be in a pharmaceutically acceptable carrier, excipient, or vehicle.

A cyclohexanehexol compound or medicament comprising a cyclohexanehexol compound can be administered to a patient by any route effective to treat an ocular disease.

The invention additionally provides a method of preparing a stable medicament comprising one or more cyclohexanehexol compound in a therapeutically effective amount for treating an ocular disease. After medicaments have been prepared, they can be placed in an appropriate container and labeled for treatment of an ocular disease. For administration of a medicament of the invention, such labeling would include amount, frequency, and method of administration.

The invention also contemplates the use of at least one cyclohexanehexol compound for treating an ocular disease or for the preparation of a medicament for treating an ocular disease. The invention additionally provides uses of a cyclohexanehexol for the prevention of an ocular disease or in the preparation of medicaments for the prevention of an ocular disease. The medicament may be in a form for consumption by a subject such as a pill, tablet, caplet, soft and hard gelatin capsule, lozenge, sachet, cachet, vegicap, liquid drop, elixir, suspension, emulsion, solution, syrup, aerosol (as a solid or in a liquid medium) sterile injectable solution, and/or sterile packaged powder for modulation (e.g., inhibition) of amyloid, amyloid oligomerization and/or aggregate formation, deposition, accumulation, clearance and/or persistence.

The invention further provides a dietary supplement composition comprising one or more cyclohexanehexol compound or nutraceutically acceptable derivatives thereof, for treatment of an ocular disease, in particular for alleviating the symptoms of an ocular disease. In an aspect, the invention provides a dietary supplement for mammalian consumption and particularly human consumption for the purpose of improving ocular function comprising a cyclohexanehexol compound, or nutraceutically acceptable derivatives thereof. In another aspect, the invention provides a supplement comprising a cyclohexanehexol compound, or nutraceutically acceptable derivative thereof for slowing degeneration and/or death of ocular cells of individuals who have taken the supplement. A dietary supplement of the invention is preferably pleasant tasting, effectively absorbed into the body and provides substantial therapeutic effects. In an aspect, a dietary supplement of the present invention is formulated as a beverage, but may be formulated in granule, capsule or suppository form.

The invention also provides a kit comprising one or more cyclohexanehexol compound, or a medicament comprising same. In an aspect, the invention provides a kit for preventing and/or treating an ocular disease, containing a medicament comprising one or more cyclohexanehexol compound, a container, and instructions for use. The composition of the kit can further comprise a pharmaceutically acceptable carrier, excipient, or vehicle. In an aspect, the invention provides a method of promoting sales of a medicament or kit of the invention comprising the public distribution of information that administration of the medicament or kit is associated with treatment or prophylaxis of an ocular disease.

These and other aspects, features, and advantages of the present invention should be apparent to those skilled in the art from the following drawings and detailed description.

### DESCRIPTION OF THE DRAWINGS

Figure 1 shows the effect of AZD103 on Aβ₁₋₄₂secretion of VEGF in ARPE-19 cells (1.5 x 10⁴)_{.}
Figure 2 shows the effect of AZD103 on Aβ₁₋₄₂secretion of VEGF in ARPE-19 cells (1.5x10⁴).

### DETAILED DESCRIPTION OF EMBODIMENTS

All technical and scientific terms used herein have the same meaning as commonly understood by one of ordinary skill in the art to which this invention belongs. For convenience, certain terms employed in the specification, examples, and appended claims are collected here.

The recitation of numerical ranges by endpoints herein includes all numbers and fractions subsumed within that range (e.g. 1 to 5 includes 1, 1.5, 2, 2.75, 3, 3.90, 4, and 5). It is also to be understood that all numbers and fractions thereof are presumed to be modified by the term "about." The term "about" means plus or minus 0.1 to 50%, 5-50%, or 10-40%, preferably 10-20%, more preferably 10% or 15%, of the number to which reference is being made. Further, it is to be understood that "a," "an," and "the" include plural referents unless the content clearly dictates otherwise. Thus, for example, reference to "a compound" includes a mixture of two or more compounds.

The terms "administering" and "administration" refer to the process by which a therapeutically effective amount of a cyclohexanehexol compound or medicament contemplated herein is delivered, for any period of time, to a subject for prevention and/or treatment purposes. The compounds and medicaments are administered in accordance with good medical practices taking into account the subject's clinical condition, the site and method of administration, dosage, patient age, sex, body weight, and other factors known to physicians.

The term "treating" refers to reversing, alleviating, or inhibiting the progress of a disease, or one or more symptoms of such disease, to which such term applies. Treating includes the management and care of a subject at diagnosis or later. A treatment may be either performed in an acute or chronic way. Depending on the condition of the subject, the term may refer to preventing a disease, and includes preventing the onset of a disease, or preventing the symptoms associated with a disease. The term also refers to reducing the severity of a disease or symptoms associated with such disease prior to affliction with the disease. Such prevention or reduction of the severity of a disease prior to affliction refers to administration of a cyclohexanehexol compound, or medicament comprising same, to a subj ect that is not at the time of administration afflicted with the disease. "Preventing" also refers to preventing the recurrence of a disease or of one or more symptoms associated with such disease. An objective of treatment is to combat the disease and includes administration of the active compounds to prevent or delay the onset of the symptoms or complications, or alleviating the symptoms or complications, or eliminating or partially eliminating the disease. The terms "treatment" and "therapeutically," refer to the act of treating, as "treating" is defined above.

The terms "subject", "individual", or "patient" are used interchangeably herein and refer to an animal including a warm-blooded animal such as a mammal. Mammal includes without limitation any members of the Mammalia. A mammal, as a subject or patient in the present disclosure, can be from the family of Primates, Carnivora, Proboscidea, Perissodactyla, Artiodactyla, Rodentia, and Lagomorpha. Among other specific embodiments a mammal of the present invention can be *Canis familiaris* (dog), *Felis catus* (cat), *Elephas maximus* (elephant), *Equus caballus* (horse), *Sus domesticus* (pig), *Camelus dromedarious* (camel), *Cervus axis* (deer), *Giraffa camelopardalis* (giraffe), *Bos taurus* (cattle/cows), *Capra hircus* (goat), *Ovis aries* (sheep), *Mus musculus* (mouse), *Lepus brachvurus* (rabbit), *Mesocricetus auratus* (hamster), *Cavia porcellus* (guinea pig)*,Meriones unguiculatus* (gerbil), or *Homo sapiens* (human). In a particular embodiment, the mammal is a human. In other embodiments, animals can be treated, the animals can be vertebrates, including both birds and mammals. Birds suitable as subjects within the confines of the present invention include *Gallus domesticus* (chicken) and *Meleagris gallopavo* (turkey). Typical subjects for treatment include persons afflicted with or suspected of having or being pre-disposed to an ocular disease, or persons susceptible to, suffering from or that have suffered from an ocular disease. A subject may or may not have a genetic predisposition for an ocular disease. In particular aspects, a subject shows symptoms of an ocular disease. In embodiments of the invention, the subjects are suspectible to, or suffer from an ocular disease.

As utilized herein, the term "healthy subject" means a subject, in particular a mammal, having no diagnosed ocular disease or symptoms of an ocular disease.

A "beneficial effect" refers to an effect of a cyclohexanehexol compound or medicament thereof in aspects of the invention, including favorable pharmacological and/or therapeutic effects, and improved biological activity. In aspects of the invention, the beneficial effects include modulation (e.g., inhibition, reversal, or reduction) of assembly, folding, accumulation, rate of aggregation and/or clearance of amyloid β or oligomers or aggregates comprising amyloid β, in particular prevention, reduction or inhibition of amyloid β aggregation or assembly of oligomers or aggregates comprising amyloid β (e.g., drusen) in ocular cells, reversal or reduction of amyloid β or oligomers or aggregates comprising amyloid β after the onset of symptoms of an ocular disease, dissolution and/or disruption of amyloid β, or oligomers or aggregates comprising amyloid β, enhanced clearance of amyloid β, or oligomers or aggregates comprising amyloid β, reduction or inhibition of VEGF or VEGF activity, and, slowing or arrest of the progress of an ocular disease. In particular embodiments of the invention, the beneficial effects include but are not limited to the following: improved ocular function, slowing of degeneration and death of ocular cells, and slowing or arrest of the progress of an ocular disease. In embodiments of the invention, the beneficial effects include increased time to relapse in a subject receiving a conventional therapy.

In an embodiment, the beneficial effect is a "sustained beneficial effect" where the beneficial effect is sustained for a prolonged period of time after termination of treatment. A treatment can be sustained over several weeks, months or years thereby having a major beneficial impact on the severity of the disease and its complications. In aspects of the invention, a beneficial effect may be sustained for a prolonged period of at least about 2 to 4 weeks, 2 to 5 weeks, 3 to 5 weeks, 2 to 6 weeks, 2 to 8 weeks, 2 to 10 weeks, 2 to 12 weeks, 2 to 14 weeks, 2 to 16 weeks, 2 to 20 weeks, 2 to 24 weeks, 2 weeks to 12 months, 2 weeks to 18 months, 2 weeks to 24 months, or several years following treatment. The period of time a beneficial effect is sustained may correlate with the duration and timing of the treatment. A subject may be treated continuously for about or at least about 2 to 4 weeks, 2 to 6 weeks, 2 to 8 weeks, 2 to 10 weeks, 2 to 12 weeks, 2 to 14 weeks, 2 to 16 weeks, 2 weeks to 6 months, 2 weeks to 12 months, 2 weeks to 18 months, or several years, periodically or continuously.

The beneficial effect may be a statistically significant effect in terms of statistical analysis of an effect of a cyclohexanehexol compound, versus the effects without such a compound. "Statistically significant" or "significantly different" effects or levels may represent levels that are higher or lower than a standard. In embodiments of the invention, the difference may be 1.5, 2, 3, 4, 5, 6, 7, 8, 9, 10, 15, 20, 25, 50, 1-10, 1-20, 1-30 or 1-50 times higher or lower compared with the effect obtained without a cyclohexanehexol compound.

The term "pharmaceutically acceptable carrier, excipient, or vehicle" refers to a medium which does not interfere with the effectiveness or activity of an active ingredient and which is not toxic to the hosts to which it is administered. A carrier, excipient, or vehicle includes diluents, binders, adhesives, lubricants, disintegrates, bulking agents, wetting or emulsifying agents, pH buffering agents, and miscellaneous materials such as absorbants that may be needed in order to prepare a particular medicament. Examples of carriers etc. include but are not limited to saline, buffered saline, dextrose, water, glycerol, ethanol, and combinations thereof. The use of such media and agents for an active substance is well known in the art. Acceptable carriers, excipients or vehicles may be selected from any of those commercially used in the art.

"Pharmaceutically acceptable salt(s)," means a salt that is pharmaceutically acceptable and has the desired pharmacological properties. By pharmaceutically acceptable salts is meant those salts which are suitable for use in contact with the tissues of a subject or patient without undue toxicity, irritation, allergic response and the like, and are commensurate with a reasonable benefit/risk ratio. Pharmaceutically acceptable salts are described for example, in S. M. Berge, et al., J. Pharmaceutical Sciences, 1977, 66:1. Suitable salts include salts that may be formed where acidic protons in the compounds are capable of reacting with inorganic or organic bases. Suitable inorganic salts include those formed with alkali metals, e.g. sodium and potassium, magnesium, calcium, and aluminum. Suitable organic salts include those formed with organic bases such as the amine bases, e.g. ethanolamine, diethanolamine, triethanolamine, tromethamine, N-methylglucamine, and the like. Suitable salts also include acid addition salts formed with inorganic acids (e.g. hydrochloric and hydrobromic acids) and organic acids (e.g. acetic acid, citric acid, maleic acid, and the alkane- and arene-sulfonic acids such as methanesulfonic acid and benezenesulfonic acid). When there are two acidic groups present, a pharmaceutically acceptable salt may be a mono-acid-mono-salt or a di-salt; and similarly where there are more than two acidic groups present, some or all of such groups can be salified.

"Therapeutically effective amount" relates to the amount or dose of a cyclohexanehexol compound or medicament thereof, that will lead to one or more desired effects, in particular, one or more beneficial effects. A therapeutically effective amount of a substance can vary according to factors such as the disease state, age, sex, and weight of the subject, and the ability of the substance to elicit a desired response in the subject. A dosage regimen may be adjusted to provide the optimum therapeutic response (e.g. beneficial effects, more particularly sustained beneficial effects). For example, several divided doses may be administered daily or the dose may be proportionally reduced as indicated by the exigencies of the therapeutic situation.

The term "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

The term "pure" in general means better than 90%, 92%, 93%, 94%, 95%, 96%, 97%, 98% or 99% pure, and "substantially pure" means a compound synthesized such that the compound, as made available for consideration into a method or medicament of the invention, has only those impurities that can not readily nor reasonably be removed by conventional purification processes.

As used herein "nutraceutically acceptable derivative" refers to a derivative or substitute for the stated chemical species that operates in a similar manner to produce the intended effect, and is structurally similar and physiologically compatible. Examples of substitutes include without limitation salts, esters, hydrates, or complexes of the stated chemical. The substitute could also be a precursor or prodrug to the stated chemical, which subsequently undergoes a reaction *in vivo* to yield the stated chemical or a substitute thereof.

"Optional" or "optionally" means that the subsequently described event or circumstance may but need not occur, and that the description includes instances where the event or circumstance occurs and instances in which it does not occur. For example, "alkyl group optionally substituted with a halo group" means that the halo may but need not be present, and the description includes situations where the alkyl group is substituted with a halo group and situations where the alkyl group is not substituted with the halo group.

"Ocular cell" or grammatical equivalents thereof used herein, refers to an ocular cell contained within the eye, i.e. *in vivo.* Ocular cells include without limitation cells of the lens, the comea (endothelial, stromal and/or epithelial corneal cells), the iris, the retina, choroid, sclera, ciliary body, vitrous body, ocular vasculature, canal of Schlemm, ocular muscle cells, optic nerve, and other ocular sensory, motor and autonomic nerves.

A "cyclohexanehexol compound" is understood to refer to any compound, which fully or partially, directly or indirectly, provides one or more therapeutic effects, in particular beneficial effects described herein, and includes a compound of the formula I, II, III or IV described herein, or an analog or derivative thereof (e.g. functional derivative, chemical derivative or variant), salt (e.g., pharmaceutically acceptable salt), prodrug, polymorph, crystalline form, solvate or hydrate thereof. In aspects of the invention, the cyclohexanehexol compound is an inositol.

A cyclohexanehexol compound includes a functional derivative, a chemical derivative, or variant. A "functional derivative" refers to a compound that possesses an activity (either functional or structural) that is substantially similar to the activity of a cyclohexanehexol compound disclosed herein. The term "chemical derivative" describes a molecule that contains additional chemical moieties which are not normally a part of the base molecule. The term "variant" is meant to refer to a molecule substantially similar in structure and function to a cyclohexanehexol compound or a part thereof. A molecule is "substantially similar" to a cyclohexanehexol compound if both molecules have substantially similar structures or if both molecules possess similar biological activity. The term "analog" includes a molecule substantially similar in function to a cyclohexanehexol compound. An "analog" can include a chemical compound that is structurally similar to another but differs slightly in composition. Differences include without limitation the replacement of an atom or functional group with an atom or functional group of a different element. Analogs and derivatives may be identified using computational methods with commercially available computer modeling programs.

A cyclohexanehexol compound includes a pharmaceutically functional derivative. A "pharmaceutically functional derivative" includes any pharmaceutically acceptable derivative of a cyclohexanehexol compound, for example, an ester or an amide, which upon administration to a subj ect is capable of providing (directly or indirectly) a cyclohexanehexol compound or an active metabolite or residue thereof. Such derivatives are recognizable to those skilled in the art, without undue experimentation (see for example Burger's Medicinal Chemistry and Drug Discovery, 5.sup.th Edition, Vol 1: Principles and Practice, which has illustrative pharmaceutically functional derivatives).

A cyclohexanehexol compound includes crystalline forms which may exist as polymorphs. Solvates of the compounds formed with water or common organic solvents are also intended to be encompassed within the term. In addition, hydrate forms of the compounds and their salts are encompassed within this invention. Further prodrugs of compounds of cyclohexanehexol compounds are encompassed within the term.

The term "solvate" means a physical association of a compound with one or more solvent molecules or a complex of variable stoichiometry formed by a solute (for example, a compound of the invention) and a solvent, for example, water, ethanol, or acetic acid. This physical association may involve varying degrees of ionic and covalent bonding, including hydrogen bonding. In certain instances, the solvate will be capable of isolation, for example, when one or more solvent molecules are incorporated in the crystal lattice of the crystalline solid. In general, the solvents selected do not interfere with the biological activity of the solute. Solvates encompass both solution-phase and isolatable solvates. Representative solvates include hydrates, ethanolates, methanolates, and the like. Dehydrate, co-crystals, anhydrous, or amorphous forms of the cyclohexanehexol compounds are also included. The term "hydrate" means a solvate wherein the solvent molecule(s) is/are H₂O, including, mono-, di-, and various poly-hydrates thereof. Solvates can be formed using various methods known in the art.

Crystalline cyclohexanehexol compounds can be in the form of a free base, a salt, or a co-crystal. Free base compounds can be crystallized in the presence of an appropriate solvent in order to form a solvate. Acid salt cyclohexanehexol compounds (e.g. HCl, HBr, benzoic acid) can also be used in the preparation of solvates. For example, solvates can be formed by the use of acetic acid or ethyl acetate. The solvate molecules can form crystal structures via hydrogen bonding, van der Waals forces, or dispersion forces, or a combination of any two or all three forces.

The amount of solvent used to make solvates can be determined by routine testing. For example, a monohydrate of a cyclohexanehexol compound would have about 1 equivalent of solvent (H₂O) for each equivalent of a cyclohexanehexol compound. However, more or less solvent may be used depending on the choice of solvate desired.

The cyclohexanehexol compounds used in the invention may be amorphous or may have different crystalline polymorphs, possibly existing in different solvation or hydration states. By varying the form of a drug, it is possible to vary the physical properties thereof. For example, crystalline polymorphs typically have different solubilities from one another, such that a more thermodynamically stable polymorph is less soluble than a less thermodynamically stable polymorph. Pharmaceutical polymorphs can also differ in properties such as shelf-life, bioavailability, morphology, vapor pressure, density, color, and compressibility.

The term "prodrug" means a covalently-bonded derivative or carrier of the parent compound or active drug substance which undergoes at least some biotransformation prior to exhibiting its pharmacological effect(s). In general, such prodrugs have metabolically cleavable groups and are rapidly transformed *in vivo* to yield the parent compound, for example, by hydrolysis in blood, and generally include esters and amide analogs of the parent compounds. The prodrug is formulated with the objectives of improved chemical stability, improved patient acceptance and compliance, improved bioavailability, prolonged duration of action, improved organ selectivity, improved formulation (e.g., increased hydrosolubility), and/or decreased side effects (e.g., toxicity). In general, prodrugs themselves have weak or no biological activity and are stable under ordinary conditions. Prodrugs can be readily prepared from the parent compounds using methods known in the art, such as those described, for example, in A Textbook of Drug Design and Development, Krogsgaard-Larsen and H. Bundgaard (eds.), Gordon & Breach, 1991, particularly Chapter 5: "Design and Applications of Prodrugs"; Design of Prodrugs, H. Bundgaard (ed.), Elsevier, 1985; Prodrugs: Topical and Ocular Drug Delivery, K. B. Sloan (ed.), Marcel Dekker, 1998; Methods in Enzymology, K. Widder et al. (eds.), Vol. 42, Academic Press, 1985, particularly pp. 309 396; Burger's Medicinal Chemistry and Drug Discovery, 5th Ed., M. Wolff (ed.), John Wiley & Sons, 1995, particularly Vol. 1 and pp. 172 178 and pp. 949 982; Pro-Drugs as Novel Delivery Systems, T. Higuchi and V. Stella (eds.), Am. Chem. Soc., 1975; and Bioreversible Carriers in Drug Design, E. B. Roche (ed.), Elsevier, 1987, each of which is incorporated herein by reference in their entireties.

Examples of prodrugs include, but are not limited to esters (e.g., acetate, formate, and benzoate derivatives) and carbamates (e.g. N,N-dimethylaminocarbonyl) of hydroxy functional groups on cyclohexanehexol compounds, and the like

The cyclohexanehexol compound is a scyllo-inositol compound, in particular a pure or substantially pure scyllo-inositol compound.

A "scyllo-inositol compound" includes compounds having the structure of the formula Va or Vb:

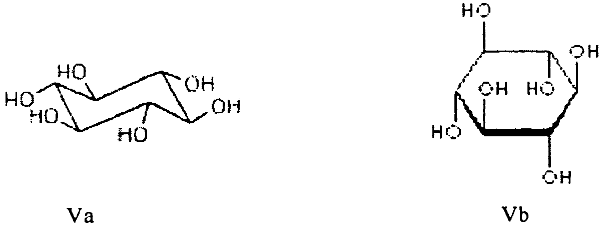

A scyllo-inositol compound includes a compound of the formula Va or Vb wherein one to six, one to five, one, two, three or four, preferably one, two or three, more preferably one or two hydroxyl groups are replaced by substituents, in particular univalent substituents, with retention of configuration. In aspects of the invention, a scyllo-inositol compound comprises a compound of the formula Va or Vb wherein one, two, three, four, five or six, preferably one or two, most preferably one, hydroxyl groups are replaced by univalent substituents, with retention of configuration. Suitable substituents include without limitation hydrogen; alkyl; substituted alkyl; acyl; alkenyl; substituted alkenyl; alkynyl; substituted alkynyl; cycloalkyl; substituted cycloalkyl; alkoxy; substituted alkoxy; aryl; aralkyl; substituted aryl; halogen; thiol; -NHR⁴¹ wherein R⁴¹ is hydrogen, acyl, alkyl or -R⁴²R⁴³ wherein R⁴² and R⁴³ are the same or different and represent acyl or alkyl; -PO₃H₂; -SR⁴⁴ wherein R⁴⁴ is hydrogen, alkyl, or -O₃H; or -OR⁴⁵ wherein R⁴⁵ is hydrogen, alkyl, or -SO₃H.

In aspects of the invention, a scyllo-inositol compound does not include scyllo-cyclohexanehexol substituted with one or more phosphate group.

Particular aspects of the invention utilize scyllo-inositol compounds of the formula Va or Vb wherein one or more of the hydroxyl groups is replaced with alkyl, in particular C₁-C₄ alkyl, more particularly methyl; acyl; chloro or fluoro; alkenyl; -NHR⁴¹ wherein R⁴¹ is hydrogen, acyl, alkyl or -R⁴²R⁴³ wherein R⁴² and R⁴³ are the same or different and represent acyl or alkyl; -SR⁴⁴wherein R⁴⁴ is hydrogen, alkyl, or -O₃H; and -OR⁴⁵ wherein R⁴⁵ is hydrogen, alkyl, or -SO₃H, more particularly -SR⁴⁴wherein R⁴⁴ is hydrogen, alkyl, or -O₃H or -OR⁴⁵ wherein R⁴⁵ is -SO₃H.

Particular aspects of the invention utilize scyllo-inositol compounds of the formula Va or Vb wherein one or more of the hydroxyl groups is replaced with alkyl; substituted alkyl; acyl; alkenyl; substitututed alkenyl; -NHR⁴¹ wherein R⁴¹ is hydrogen, acyl, alkyl, or -R⁴²R⁴³ wherein R⁴² and R⁴³ are the same or different and represent acyl or alkyl; -SR⁴⁴ wherein R⁴⁴ is hydrogen, alkyl, or -O₃H; or -OR⁴⁵ wherein R⁴⁵ is hydrogen, alkyl or -SO₃H.

Particular aspects of the invention utilize scyllo-inositol compounds of the formula Va or Vb wherein one or more of the hydroxyl groups is replaced with alkyl; substituted alkyl; acyl; alkenyl; substituted alkenyl; alkynyl; substituted alkynyl; alkoxy; substituted alkoxy; halogen; thiol; -NHR⁴¹ wherein R⁴¹ is hydrogen, acyl, alkyl or -R⁴²R⁴³ wherein R⁴² and R⁴³ are the same or different and represent acyl or alkyl; -PO₃H₂; -SR⁴⁴ wherein R⁴⁴ is hydrogen, alkyl, or -O₃H; -OR⁴⁵ wherein R⁴⁵ is hydrogen, alkyl, or -OR⁴⁵ wherein R⁴⁵ is -SO₃H.

Particular aspects of the invention utilize scyllo-inositol compounds of the formula Va or Vb wherein one or more of the hydroxyl groups is replaced with alkyl; substituted alkyl; acyl; alkenyl; substituted alkenyl; alkynyl; substituted alkynyl; alkoxy; substituted alkoxy; halogen; or thiol.

Particular aspects of the invention utilize scyllo-inositol compounds of the formula Va or Vb wherein one of the hydroxyl groups is replaced with alkyl, in particular C₁-C₄ alkyl, more particularly methyl.

Particular aspects of the invention utilize scyllo-inositol compounds of the formula Va or Vb wherein one of the hydroxyl groups is replaced with alkoxy, in particular C₁-C₄ alkoxy, more particularly methoxy or ethoxy, most particularly methoxy.

Particular aspects of the invention utilize scyllo-inositol compounds of the formula Va or Vb wherein one of the hydroxyl groups is replaced with halogen, in particular chloro or fluoro, more particularly fluoro.

Particular aspects of the invention utilize scyllo-inositol compounds of the formula Va or Vb wherein one of the hydroxyl groups is replaced with thiol.

In embodiments of the invention, the scyllo-inositol compound designated AZD-103/ELND005 (Elan Corporation) is used in the formulations, dosage forms, methods and uses disclosed herein.

In embodiments of the invention, the cyclohexanehexol is *O*-methyl-scyllo-inositol

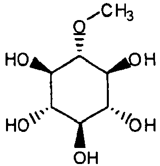

In embodiments of the invention, the cyclohexanehexol is 1-chloro-1-deoxy-scyllo-inositol.

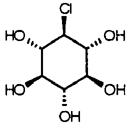

In aspects of the invention, the cyclohexanehexol is an epi-inositol compound, in particular a pure or substantially pure epi-inositol compound.

An "epi-inositol compound" includes compounds having the base structure of formula VI:

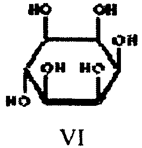

An epi-inositol compound includes a compound of the formula VI wherein one to six, one to five, one, two, three or four, preferably one, two or three, more preferably one or two hydroxyl groups are replaced by substituents, in particular univalent substituents, with retention of configuration. In aspects of the invention, an epi-inositol compound comprises a compound of the formula VI wherein one, two, three, four, five or six, preferably one or two, most preferably one, hydroxyl groups are replaced by univalent substituents, with retention of configuration. Suitable substituents include without limitation hydrogen; alkyl; substituted alkyl; acyl; alkenyl; substituted alkenyl; alkynyl; substituted alkynyl; cycloalkyl; substituted cycloalkyl; alkoxy; substituted alkoxy; aryl; aralkyl; substituted aryl; halogen; thiol; -NHR⁴¹ wherein R⁴¹ is hydrogen, acyl, alkyl or -R⁴²R⁴³ wherein R⁴² and R⁴³ are the same or different and represent acyl or alkyl; -PO₃H₂; -SR⁴⁴ wherein R⁴⁴ is hydrogen, alkyl, or -O₃H; or -OR⁴⁵ wherein R⁴⁵ is hydrogen, alkyl, or -SO₃H.

Particular aspects of the invention utilize epi-inositol compounds of the formula VI wherein one or more of the hydroxyl groups is replaced with alkyl, in particular C₁-C₄ alkyl, more particularly methyl; acyl; chloro or fluoro; alkenyl; -NHR⁴¹ wherein R⁴¹ is hydrogen, acyl, alkyl or -R⁴²R⁴³ wherein R⁴² and R⁴³ are the same or different and represent acyl or alkyl; -SR⁴⁴ wherein R⁴⁴ is hydrogen, alkyl, or -O₃H; and -OR⁴⁵ wherein R⁴⁵ is hydrogen, alkyl, or -SO₃H, more particularly -SR⁴⁴ wherein R⁴⁴ is hydrogen, alkyl, or -O₃H or -OR⁴⁵ wherein R⁴⁵ is -SO₃H.

Particular aspects of the invention utilize epi-inositol compounds of the formula VI wherein one or more of the hydroxyl groups is replaced with alkyl; substituted alkyl; acyl; alkenyl; substitututed alkenyl; -NHR⁴¹ wherein R⁴¹ is hydrogen, acyl, alkyl, or -R⁴²R⁴³ wherein R⁴² and R⁴³ are the same or different and represent acyl or alkyl; -SR⁴⁴ wherein R⁴⁴ is hydrogen, alkyl, or -O₃H; or -OR⁴⁵ wherein R⁴⁵ is hydrogen, alkyl or -SO₃H.

Particular aspects of the invention utilize epi-inositol compounds of the formula VI wherein one or more of the hydroxyl groups is replaced with alkyl; substituted alkyl; acyl; alkenyl; substituted alkenyl; alkynyl; substituted alkynyl; alkoxy; substituted alkoxy; halogen; thiol; -NHR⁴¹ wherein R⁴¹ is hydrogen, acyl, alkyl or -R⁴²R⁴³ wherein R⁴² and R⁴³ are the same or different and represent acyl or alkyl; -PO₃H₂; -SR⁴⁴ wherein R⁴⁴ is hydrogen, alkyl, or -O₃H; -OR⁴⁵ wherein R⁴⁵ is hydrogen, alkyl, or -OR⁴⁵ wherein R⁴⁵ is -SO₃H.

Particular aspects of the invention utilize epi-inositol compounds of the formula VI wherein one or more of the hydroxyl groups is replaced with alkyl; substituted alkyl; acyl; alkenyl; substituted alkenyl; alknyl; substituted alkynyl; alkoxy; substituted alkoxy; halogen; or thiol.

Particular aspects of the invention utilize epi-inositol compounds of the formula VI wherein one of the hydroxyl groups is replaced with alkyl, in particular C₁-C₄ alkyl, more particularly methyl.

Particular aspects of the invention utilize epi-inositol compounds of the formula VI wherein one of the hydroxyl groups is replaced with alkoxy, in particular C₁-C₄ alkoxy, more particularly methoxy or ethoxy, most particularly methoxy.

Particular aspects of the invention utilize epi-inositol compounds of the formula VI wherein one of the hydroxyl groups is replaced with halogen, in particular chloro or fluoro, more particularly fluoro.

Particular aspects of the invention utilize epi-inositol compounds of the formula VI wherein one of the hydroxyl groups is replaced with thiol.

In aspects of the invention, the cyclohexanehexol is epi-inositol, in particular a pure or substantially pure epi-inositol.

Cyclohexanehexol compounds utilized in the invention may be prepared using reacti ons and methods generally known to the person of ordinary skill in the art, having regard to that knowledge and the disclosure of this application. The reactions are performed in a solvent appropriate to the reagents and materials used and suitable for the reactions being effected. It will be understood by those skilled in the art of organic synthesis that the functionality present on the compounds should be consistent with the proposed reaction steps. This will sometimes require modification of the order of the synthetic steps or selection of one particular process scheme over another in order to obtain a desired compound of the invention. It will also be recognized that another major consideration in the development of a synthetic route is the selection of the protecting group used for protection of the reactive functional groups present in the compounds described in this invention. An authoritative account describing the many alternatives to the skilled artisan is Greene and Wuts (Protective Groups In Organic Synthesis, Wiley and Sons, 1991).

The starting materials and reagents used in preparing cyclohexanehexol compounds are either available from commercial suppliers such as the Aldrich Chemical Company (Milwaukee, Wis.), Bachem (Torrance, Calif.), Sigma (St. Louis, Mo.), or Lancaster Synthesis Inc. (Windham, N.H.) or are prepared by methods well known to a person of ordinary skill in the art, following procedures described in such references as Fieser and Fieser's Reagents for Organic Synthesis, vols. 1-17, John Wiley and Sons, New York, N.Y., 1991; Rodd's Chemistry of Carbon Compounds, vols. 1-5 and supps., Elsevier Science Publishers, 1989; Organic Reactions, vols. 1-40, John Wiley and Sons, New York, N.Y., 1991; March J.: Advanced Organic Chemistry, 4th ed., John Wiley and Sons, New York, N.Y.; and Larock: Comprehensive Organic Transformations, VCH Publishers, New York, 1989.

The starting materials, intermediates, and cyclohexanehexol compounds may be isolated and purified using conventional techniques, such as precipitation, filtration, distillation, crystallization, chromatography, and the like. The compounds may be characterized using conventional methods, including physical constants and spectroscopic methods, in particular HPLC.

Cyclohexanehexol compounds which are basic in nature can form a wide variety of different salts with various inorganic and organic acids. In practice it is desirable to first isolate a cyclohexanehexol compound from the reaction mixture as a pharmaceutically unacceptable salt and then convert the latter to the free base compound by treatment with an alkaline reagent and subsequently convert the free base to a pharmaceutically acceptable acid addition salt. The acid addition salts of the base compounds are readily prepared by treating the base compound with a substantially equivalent amount of the chosen mineral or organic acid in an aqueous solvent medium or in a suitable organic solvent such as methanol or ethanol. Upon careful evaporation of the solvent, the desired solid salt is obtained.

Cyclohexanehexol compounds which are acidic in nature are capable of forming base salts with various pharmacologically acceptable cations. These salts may be prepared by conventional techniques by treating the corresponding acidic compounds with an aqueous solution containing the desired pharmacologically acceptable cations and then evaporating the resulting solution to dryness, preferably under reduced pressure. Alternatively, they may be prepared by mixing lower alkanolic solutions of the acidic compounds and the desired alkali metal alkoxide together and then evaporating the resulting solution to dryness in the same manner as before. In either case, stoichiometric quantities of reagents are typically employed to ensure completeness of reaction and maximum product yields.

Scyllo-inositol compounds can be prepared using conventional processes or they may be obtained from commercial sources. For example, scyllo-inositol compounds can be prepared using chemical and/or microbial processes. In aspects of the invention, a scyllo-inositol is produced using process steps described by M. Sarmah and Shashidhar, M., Carbohydrate Research, 2003, 338, 999-1001, Husson, C., et al, Carbohyrate Research 307 (1998) 163-165; Anderson R. and E.S. Wallis, J. American Chemical Society (US), 1948, 70:2931-2935; Weissbach, A., J Org Chem (US), 1958, 23:329-330; Chung, S.K. et al., Bioorg Med Chem. 1999, 7(11):2577-89; or Kiely D.E., and Fletcher, H.G., J. American Chemical Society (US) 1968, 90:3289-3290; described in JP09-140388, DE 3,405,663 (Merck Patent GMBH), JP04-126075, JP05-192163, or WO06109479, or described in WO0503577, US20060240534, EP1674578, JP9140388, JP09140388, JP02-184912, JP03-102492 (Hokko Chemical Industries). In particular aspects of the compositions and methods of the invention, a scyllo-inositol is prepared using the chemical process steps described in Husson, C., et al, Carbohydrate Research 307 (1998) 163-165. In other aspects of the compositions and methods of the invention, a scyllo-inositol is prepared using microbial process steps similar to those described in WO05035774 (EP1674578 and US20060240534) JP2003102492, or JP09140388 (Hokko Chemical Industries). Derivatives may be produced by introducing substituents into a scyllo-inositol compound using methods well known to a person of ordinary skill in the art.

Epi-inositol compounds can be prepared using conventional processes or they may be obtained from commercial sources. In aspects of the invention, an epi-inositol compounds can be prepared using chemical and/or microbial processes. For example, an epi-inositol may be prepared by the process described by V. Pistarà (Tetrahedron Letters 41, 3253, 2000), Magasanik B., and ChargaffE. (J Biol Chem, 1948, 174:173188), US Patent No. 7,157,268, or in PCT Published Application No. WO0075355. Derivatives may be produced by introducing substituents into an epi-inositol compound using methods well known to a person of ordinary skill in the art.

A cyclohexanehexol compound may additionally comprise a carrier, including without limitation one or more of a polymer, carbohydrate, peptide or derivative thereof. A carrier may be substituted with substituents described herein including without limitation one or more alkyl, amino, nitro, halogen, thiol, thioalkyl, sulfate, sulfonyl, sulfenyl, sulfinyl, sulfoxide, hydroxyl groups. A carrier can be directly or indirectly covalently attached to a compound of the invention. In aspects of the invention the carrier is an amino acid including alanine, glycine, proline, methionine, serine, threonine, or asparagine. In other aspects the carrier is a peptide including alanyl-alanyl, prolyl-methionyl, or glycyl-glycyl.

A carrier also includes a molecule that targets a compound of the invention to a particular tissue or organ. In particular, a carrier may facilitate or enhance transport of a compound of the invention to the brain by either active or passive transport.

A "polymer" as used herein refers to molecules comprising two or more monomer subunits that may be identical repeating subunits or different repeating subunits. A monomer generally comprises a simple structure, low-molecular weight molecule containing carbon. Polymers can be optionally substituted. Examples of polymers which can be used in the present invention are vinyl, acryl, styrene, carbohydrate derived polymers, polyethylene glycol (PEG), polyoxyethylene, polymethylene glycol, poly-trimethylene glycols, polyvinylpyrrolidone, polyoxyethylene-polyoxypropylene block polymers, and copolymers, salts, and derivatives thereof. In particular aspects of the invention, the polymer is poly(2-acrylamido-2-methyl-1-propanesulfonic acid), poly(2-acrylamido-2-methyl,-1-propanesulfonic acid-coacrylonitrile, poly(2-acrylamido-2-methyl-1-propanesulfonic acid-co-styrene), poly(vinylsulfonic acid), poly(sodium 4-styrenesulfonic acid), and sulfates and sulfonates derived therefrom; poly(acrylic acid), poly(methylacrylate), poly(methyl methacrylate), and poly(vinyl alcohol).

A "carbohydrate" as used herein refers to a polyhydroxyaldehyde, or polyhydroxyketone and derivatives thereof. The simplest carbohydrates are monosaccharides, which are small straight-chain aldehydes and ketones with many hydroxyl groups added, usually one on each carbon except the functional group. Examples of monosaccharides include erythrose, arabinose, allose, altrose, glucose, mannose, threose, xylose, gulose, idose, galactose, talose, aldohexose, fructose, ketohexose, ribose, and aldopentose. Other carbohydrates are composed of monosaccharide units, including disaccharides, oligosaccharides, or polysaccharides, depending on the number of monosaccharide units. Disaccharides are composed of two monosaccharide units joined by a covalent glycosidic bond. Examples of disaccharides are sucrose, lactose, and maltose. Oligosaccharides and polysaccharides, are composed of longer chains of monosaccharide units bound together by glycosidic bonds. Oligosaccharides generally contain between 3 and 9 monosaccharide units and polysaccharides contain greater than 10 monosaccharide units. A carbohydrate group may be substituted at one two, three or four positions, other than the position of linkage to a compound of the formula I, II, III or IV. For example, a carbohydrate may be substituted with one or more alkyl, amino, nitro, halo, thiol, carboxyl, or hydroxyl groups, which are optionally substituted. Illustrative substituted carbohydrates are glucosamine or galactosamine.

In aspects of the invention, the carbohydrate is a sugar, in particular a hexose or pentose and may be an aldose or a ketose. A sugar may be a member of the D or L series and can include amino sugars, deoxy sugars, and their uronic acid derivatives. In embodiments of the invention where the carbohydrate is a hexose, the hexose is selected from the group consisting of glucose, galactose, or mannose, or substituted hexose sugar residues such as an amino sugar residue such as hexosamine, galactosamine, glucosamine, in particular D-glucosamine (2-amino-2-doexy-D-glucose) or D-galactosamine (2-amino-2-deoxy-D-galactose). Suitable pentose sugars include arabinose, fucose, and ribose.

A sugar residue may be linked to a cyclohexanehexol compound from a 1,1 linkage, 1,2 linkage, 1,3 linkage, 1,4 linkage, 1,5 linkage, or 1,6 linkage. A linkage may be via an oxygen atom of a cyclohexanehexol compound. An oxygen atom can be replaced one or more times by -CH₂- or -S- groups.

The term "carbohydrate" also includes glycoproteins such as lectins (e.g. concanavalin A, wheat germ agglutinin, peanutagglutinin, seromucoid, and orosomucoid) and glycolipids such as cerebroside and ganglioside.

A "peptide" for use as a carrier in the practice of the present invention includes one, two, three, four, or five or more amino acids covalently linked through a peptide bond. A peptide can comprise one or more naturally occurring amino acids, and analogs, derivatives, and congeners thereof. A peptide can be modified to increase its stability, bioavailability, solubility, etc. "Peptide analogue" and "peptide derivative" as used herein include molecules which mimic the chemical structure of a peptide and retain the functional properties of the peptide. In aspects of the invention the carrier is an amino acid such as alanine, glycine, proline, methionine, serine, threonine, histidine, or asparagine. In other aspects the carrier is a peptide such as alanyl-alanyl, prolyl-methionyl, or glycyl-glycyl. In still other aspects, the carrier is a polypeptide such as albumin, antitrypsin, macroglobulin, haptoglobin, caeruloplasm, transferrin, α- or β- lipoprotein, β- or γ- globulin or fibrinogen.

Approaches to designing peptide analogues, derivatives and mimetics are known in the art. For example, see Farmer, P. S. in Drug Design (E. J. Ariens, ed.) Academic Press, New York, 1980, vol. 10, pp. 119-143; Ball. J. B. and Alewood, P. F. (1990) J Mol. Recognition 3:55; Morgan, B. A. and Gainor, J. A. (1989) Ann. Rep. Med. Chem. 24:243; and Freidinger, R. M. (1989) Trends Pharmacol. Sci. 10:270. See also Sawyer, T. K. (1995) "Peptidomimetic Design and Chemical Approaches to Peptide Metabolism" in Taylor, M. D. and Amidon, G. L. (eds.) Peptide-Based Drug Design: Controlling Transport and Metabolism, Chapter 17; Smith, A. B. 3rd, et al. (1995) J. Am. Chem Soc. 117:11113-11123; Smith, A. B. 3rd, et al. (1994) J. Am. Chem. Soc. 116:9947-9962; and Hirschman, R., et al. (1993) J. Am. Chem. Soc. 115:12550-12568.

Examples of peptide analogues, derivatives and peptidomimetics include peptides substituted with one or more benzodiazepine molecules (see e.g., James, G. L. et al. (1993) Science 260:1937-1942), peptides with methylated amide linkages and "retro-inverso" peptides (see U.S. Pat. No. 4,522,752 by Sisto).

Examples of peptide derivatives include peptides in which an amino acid side chain, the peptide backbone, or the amino- or carboxy-terminus has been derivatized (e.g., peptidic compounds with methylated amide linkages).

The term mimetic, and in particular, peptidomimetic, is intended to include isosteres. The term "isostere" refers to a chemical structure that can be substituted for a second chemical structure because the steric conformation of the first structure fits a binding site specific for the second structure. The term specifically includes peptide back-bone modifications (i.e., amide bond mimetics) well known to those skilled in the art. Such modifications include modifications of the amide nitrogen, the alpha-carbon, amide carbonyl, complete replacement of the amide bond, extensions, deletions or backbone crosslinks. Other examples of isosteres include peptides substituted with one or more benzodiazepine molecules (see e.g., James, G. L. et al. (1993) Science 260:1937-1942)

Other possible modifications include an N-alkyl (or aryl) substitution ([CONR]), backbone crosslinking to construct lactams and other cyclic structures, substitution of all D-amino acids for all L-amino acids within the compound ("inverso" compounds) or retro-inverso amino acid incorporation ([NHCO]). By "inverso" is meant replacing L-amino acids of a sequence with D-amino acids, and by "retro-inverso" or "enantio-retro" is meant reversing the sequence of the amino acids ("retro") and replacing the L-amino acids with D-amino acids. For example, if the parent peptide is Thr-Ala-Tyr, the retro modified form is Tyr-Ala-Thr, the inverso form is thr-ala-tyr, and the retro-inverso form is tyr-ala-thr (lower case letters refer to D-amino acids). Compared to the parent peptide, a retro-inverso peptide has a reversed backbone while retaining substantially the original spatial conformation of the side chains, resulting in a retro-inverso isomer with a topology that closely resembles the parent peptide. See Goodman et al. "Perspectives in Peptide Chemistry" pp. 283-294 (1981). See also U.S. Pat. No. 4,522,752 by Sisto for further description of "retro-inverso" peptides.

A peptide can be attached to a compound of the invention through a functional group on the side chain of certain amino acids (e.g. serine) or other suitable functional groups. In embodiments of the invention the carrier may comprise four or more amino acids with groups attached to three or more of the amino acids through functional groups on side chains. In another embodiment, the carrier is one amino acid, in particular a sulfonate derivative of an amino acid, for example cysteic acid.

The ocular disease is a macular degeneration-related disorder. The term "macular degeneration-related disorder" includes any of a number of conditions in which the retinal macula degenerates or becomes dysfunctional, e.g., as a consequence of decreased growth of cells of the macula, increased death or rearrangement of the cells of the macula (e.g., retinal pigment epithelium cells), loss of normal biological function, or a combination of these events. Macular degeneration results in the loss of integrity of the histoarchitecture of the cells and/or extracellular matrix of the macula and/or the loss of function of macula cells. Examples of macular degeneration-related disorders include, without limitation, age-related macular degeneration, North Carolina macular dystrophy, Sorsby's fundus dystrophy, Stargardt's disease, pattern dystrophy, Best disease, dominant drusen, and malattia leventinese (radial drusen). The term also includes extramacular changes that occur prior to, or following dysfunction and/or degeneration of the macula. Thus, the term also broadly encompasses any condition which alters or damages the integrity or function of the macula (e.g., damage to the retinal pigment epithelium or Bruch's membrane). By way of example, the term includes retinal detachment, chorioretinal degenerations, retinal degenerations, photoreceptor degenerations, retinal pigment epithelium degenerations, mucopolysaccharidoses, rod-cone dystrophies, cone-rod dystrophies and cone degenerations.

In an embodiment, the ocular disease is age-related macular degeneration.

In a particular embodiment, the ocular disease is central geographic atrophy, non-neovascular or the dry form of age-related macular degeneration.

In a particular embodiment, the ocular disease is neovascular, exudative or the wet form of age-related macular degeneration, in particular the classic or occult type (i.e., classic choroidal neovascularization and occult choroidal neovascularization).

### Medicaments

A cyclohexanehexol compound or salts thereof as an active ingredient can be directly administered to a patient, but it is preferably administered as a preparation in the form of a medicament containing the active ingredient and pharmaceutically acceptable carriers, excipients, and vehicles. Therefore, the invention contemplates a medicament comprising a therapeutically effective amount of an isolated, in particular pure, cyclohexanehexol compound, more particularly a scyllo-cyclohexanehexol compound or analog or derivative thereof, for treating an ocular disease or symptoms caused by an ocular disease, suppressing the progression of an ocular disease, and/or providing beneficial effects.

Medicaments of the present invention or fractions thereof comprise suitable pharmaceutically acceptable carriers, excipients, and vehicles selected based on the intended form of administration, and consistent with conventional pharmaceutical practices. Suitable pharmaceutical carriers, excipients, and vehicles are described in the standard text, Remington: The Science and Practice of Pharmacy. (21 st Edition, Popovich, N (eds), Advanced Concepts Institute, University of the Sciences in Philadelphia, Philadelphia, PA. 2005). A medicament of the invention can be in any form suitable for administration to a patient including a liquid solution, suspension, emulsion, tablet, pill, capsule, sustained release formulation, or powder.

Examples of preparations which are appropriate for oral administration can include capsules, tablets, powders, fine granules, solutions and syrups, where the active components can be combined with an oral, non-toxic pharmaceutically acceptable inert carrier such as lactose, starch, sucrose, cellulose, methyl cellulose, magnesium stearate, glucose, calcium sulfate, dicalcium phosphate, sodium saccharine, magnesium carbonate mannitol, sorbital, and the like. For oral administration in a liquid form, the active components may be combined with any oral, non-toxic, pharmaceutically acceptable inert carrier such as ethanol, glycerol, water, and the like. Suitable binders (e.g. gelatin, starch, com sweeteners, natural sugars including glucose, natural and synthetic gums, and waxes), lubricants (e.g. sodium oleate, sodium stearate, magnesium stearate, sodium benzoate, sodium acetate, and sodium chloride), disintegrating agents (e.g. starch, methyl cellulose, agar, bentonite, and xanthan gum), flavoring agents, and coloring agents may also be combined in the medicaments or components thereof. Medicaments as described herein can further comprise wetting or emulsifying agents, or pH buffering agents.

Medicaments which are appropriate for parenteral administration may include aqueous solutions, syrups, aqueous or oil suspensions and emulsions with edible oil such as cottonseed oil, coconut oil or peanut oil. In aspects of the invention medicaments for parenteral administration include sterile aqueous or non-aqueous solvents, such as water, isotonic saline, isotonic glucose solution, buffer solution, or other solvents conveniently used for parenteral administration of therapeutically active agents. Dispersing or suspending agents that can be used for aqueous suspensions include synthetic or natural gums, such as tragacanth, alginate, acacia, dextran, sodium carboxymethylcellulose, gelatin, methylcellulose, and polyvinylpyrrolidone. A medicament intended for parenteral administration may also include conventional additives such as stabilizers, buffers, or preservatives, e.g. antioxidants such as methylhydroxybenzoate or similar additives.

Examples of additives for medicaments that can be used for injection or drip include a resolvent or a solubilizerthat can compose an aqueous injection or an injection to be dissolved before use, such as distilled water for injection, physiological saline and propylene glycol, isotonizing agents such as glucose, sodium chloride, D-mannitol, and glycerine, and pH modifiers such as inorganic acid, organic acid, inorganic bases or organic base.

A medicament can be formulated as a suppository, with traditional binders and carriers such as triglycerides. Various known delivery systems can be used to administer a medicament of the invention, e.g. encapsulation in liposomes, microparticles, microcapsules, and the like. Medicaments can also be formulated as pharmaceutically acceptable salts as described herein.

In aspects, a medicament of the invention is a solution, suspension, or emulsion (dispersion) in a suitable ophthalmic formulation, and optionally comprising an appropriate buffer system (e.g., sodium phosphate, sodium acetate, sodium citrate, or sodium borate). Formulations for intraocular or periocular administration may additionally comprise physiologically balanced irrigating solutions which are adapted to maintain the physical structure and function of the tissue during invasive or noninvasive medical procedures. A physiologically balanced irrigating solution may generally comprise electrolytes (e.g., sodium, potassium, calcium, magnesium, and/or chloride); an energy source (e.g., dextrose); and a buffer to maintain the pH of the solution at or near physiological levels. Physiologically balanced intraocular solutions are well-known and/or commercially available and include Lactated Ringers Solution, BSS® Sterile Irrigating Solution, and BSS Plus® Intraocular Irrigating Solution (Alcon Laboratories, Inc. Fort Worth, Tex).

In aspects, a medicament of the invention is an ophthalmic formulation, including a topical ophthalmic formulation. In a particular aspect, an ophthalmic formulation is provided comprising a cyclohexanehexol compound and an ophthalmologically acceptable carrier, excipient, or vehicle. An ophthalmic formulation may comprise ophthalmologically acceptable preservatives, surfactants, viscosity enhancers, buffers, sodium chloride and/or water to form aqueous sterile ophthalmalic solutions and suspensions. An ophthalmic gel formulation is also contemplated comprising a cyclohexanehexol compound and a hydrophilic base (e.g., derived from carboxyvinyl polymers such as Carbopol® (BF Goodrich Company), and optionally preservatives and tonicity agents.

A medicament can be sterilized by, for example, filtration through a bacteria retaining filter, addition of sterilizing agents to the medicament, irradiation of the medicament, or heating the medicament. Alternatively, the medicaments may be provided as sterile solid preparations e.g., lyophilized powder, which are readily dissolved in sterile solvent immediately prior to use.

After medicaments have been prepared, they can be placed in an appropriate container and labeled for treatment of an indicated condition (i.e., an ocular disease). For administration of a medicament, such labeling would include amount, frequency, and method of administration.

A cyclohexanhexol compound may be in a form suitable for administration as a dietary supplement. A supplement may optionally include inactive ingredients such as diluents or fillers, viscosity-modifying agents, preservatives, flavorings, colorants, or other additives conventional in the art. By way of example only, conventional ingredients such as beeswax, lecithin, gelatin, glycerin, caramel, and carmine may be included. A dietary supplement composition may optionally comprise a second active ingredient such as pinitol or an active derivative or metabolite thereof.

A dietary supplement may be provided as a liquid dietary supplement e.g., a dispensable liquid) or alternatively the compositions may be formulated as granules, capsules or suppositories. The liquid supplement may include a number of suitable carriers and additives including water, glycols, oils, alcohols, flavoring agents, preservatives, coloring agents and the like. In capsule, granule or suppository form, the dietary compositions are formulated in admixture with a pharmaceutically acceptable carrier.

A supplement may be presented in the form of a softgel which is prepared using conventional methods. A softgel typically includes a layer of gelatin encapsulating a small quantity of the supplement. A supplement may also be in the form of a liquid-filled and sealed gelatin capsule, which may be made using conventional methods.

To prepare a dietary supplement composition in capsule, granule or suppository form, one or more compositions comprising cyclohexanehexol compounds may be intimately admixed with a pharmaceutically acceptable carrier according to conventional formulation techniques. For solid oral preparations such as capsules and granules, suitable carriers and additives such as starches, sugars, diluents, granulating agents, lubricants, binders, disintegrating agents and the like may be included.

According to the invention, a kit is provided. In an aspect, the kit comprises a cyclohexanehexol compound or a medicament of the invention in kit form. The kit can be a package which houses a container which contains a cyclohexanehexol compound or medicament of the invention and also houses instructions for administering the cyclohexanehexol compound or medicament to a subject. The invention further relates to a commercial package comprising a cyclohexanehexol compound or medicament together with instructions for simultaneous, separate or sequential use. In particular, a label may include amount, frequency and method of administration.

In embodiments of the invention, a pharmaceutical pack or kit is provided comprising one or more containers filled with one or more of the ingredients of a medicament of the invention to provide a beneficial effect, in particular a sustained beneficial effect. Associated with such container(s) can be various written materials such as instructions for use, or a notice in the form prescribed by a governmental agency regulating the labeling, manufacture, use or sale of pharmaceuticals or biological products, which notice reflects approval by the agency of manufacture, use, or sale for human administration.

The invention also relates to articles of manufacture and kits containing materials useful for treating ocular diseases. An article of manufacture may comprise a container with a label. Examples of suitable containers include bottles, vials, and test tubes which may be formed from a variety of materials including glass and plastic. A container holds a medicament or formulation of the invention comprising a cyclohexanehexol compound which is effective for treating an ocular disease. The label on the container indicates that the medicament of formulation is used for treating ocular diseases such as macular degeneration and may also indicate directions for use. The container may also be adapted for administration of the composition to the eye, such as a bottle for eyedrops. A container or unit dosage may also be adapted for implantation or injection in to the eye or tissues surrounding the eye such as the periocular tissue. In aspects of the invention, a medicament or formulation in a container may comprise any of the ophthalmic medicaments or formulations disclosed herein.

The invention also contemplates kits comprising any one or more of a cyclohexanehexol compound. In aspects of the invention, a kit of the invention comprises a container described herein. In particular aspects, a kit of the invention comprises a container described herein and a second container comprising a buffer. A kit may additionally include other materials desirable froma commercial and user standpoint, including, without limitation, buffers, diluents, filters, needles, syringes, and package inserts with instructions for performing any methods disclosed herein (e.g., methods for treating ocular diseases such as glaucoma or macular degeneration). A medicament or formulation in a kit of the invention may comprise any of the ophthalmic formulations or compositions disclosed herein.

In aspects of the invention, the kits may be useful for any of the methods disclosed herein, including, without limitation treating a subject suffering from an ocular disease (e.g., glaucoma or macular degeneration). Kits of the invention may contain instructions for practicing any of the methods described herein.

### Treatment Methods

The invention contemplates the use of therapeutically effective amounts of a cyclohexanehexol compound or medicament of the invention for treating an ocular disease, in particular preventing, and/or ameliorating disease severity, disease symptoms, and/or periodicity of recurrence of an ocular disease. The invention also contemplates treating in mammals an ocular disease using the medicaments or treatments of the invention. Such uses and treatments may be effective for retarding the effects of an ocular disease, including specifically, but not exclusively, degeneration of ocular cells and/or ocular function.

According to the invention, a cyclohexanehexol compound may be administered to any subject in the general population as prophylaxis against the possibility that the person may in the future develop an ocular disease. In particular embodiments, a cyclohexanehexol compound may be administered to a subject suspected of being at risk for an ocular disease, for example, by virtue of being in a family with a higher than normal incidence of an ocular disease or due to a defined genetic proclivity. Another category of subjects who may, in particular embodiments of the invention be prophylactically treated with a cyclohexanehexol compound, are persons who have experienced an environmental exposure believed to be associated with the development of an ocular disease such as exposure to pesticides, herbicides, organic solvents, mercury, lead, etc.

In an aspect, the invention provides use of a cyclohexanehexol compound or medicament of the invention to prophylactically treat persons in the general population and more particularly persons believed to be at risk for developing an ocular disease because of, for example, a positive family history for the disease and/or the presence of a genetic defect. In addition, a cyclohexanehexol compound or a medicament of the invention may be used to treat persons already diagnosed with an ocular disease (e.g. AMD) to delay the progression of existing ocular impairment and/or to delay the onset of not yet detected ocular impairment.

In addition a cyclohexanehexol compound may be administered to a subject in the early stages of an ocular disease (e.g. AMD), in particular upon a determination that the diagnosis of an ocular disease is probable. A period considered an "early stage" can be the first 6, 8, or 12 months after the onset of symptoms.

In aspects of the invention, a cyclohexanehexol compound may be administered to a subject in the later stages to delay the onset of symptoms. A period considered a "later stage" can be more than 12 months after the onset of symptoms.

The medicaments and treatments of the invention preferably provide beneficial effects. In an embodiment, beneficial effects of a medicament or treatment of the invention, in particular for macular degeneration related-disorder, can manifest as one or more or all of the following:
a) A reduction, slowing or prevention of an increase in, or an absence of symptoms of an ocular disease after administration to a subject with symptoms of the disease.
b) A reduction, slowing or prevention of an increase in accumulation of amyloid, or oligomers or aggregates comprising amyloid in ocular cells relative to the levels measured in the absence of a cyclohexanehexol compound or medicament disclosed herein in subjects preferably with symptoms of an oculcar disease. In aspects of the invention, the cyclohexanehexol compound or medicament induces at least about a 2%, 5%, 10%, 15%, 20%, 30%, 40%, 50%, 60%, 70%, 80%, or 90% decrease in accumulation of amyloid, or oligomers or aggregates comprising amyloid.
c) A reduction in the kinetics of assembly of oligomers and/or aggregates comprising amyloid in particular a 2%, 5%, 10%, 15%, 20%, 30%, 40%, 50%, 60%, 70%, 80%, or 90% reduction in the kinetics of assembly of such oligomer and/or aggregates.
d) A reduction, slowing or prevention of an increase in degeneration of ocular cells relative to the levels measured in the absence of a cyclohexanehexol compound or medicament disclosed herein in subjects with symptoms of an ocular disease, in particular macular degeneration. In aspects of the invention, the cyclohexanehexol compound or medicament induces at least about a 2%, 5%, 10%, 15%, 20%, 30%, 40%, 50%, 60%, 70%, 80%, or 90% decrease in degeneration of ocular cells.
e) An increase or restoration of ocular function after administration to a subject with symptoms of an ocular disease. In aspects of the invention a cyclohexanehexol compound or medicament disclosed herein induces at least about a 0.05%, 0.1%, 0.5%, 1%, 2%, 5%, 10%, 15%, 20%, 30%, 33%, 35%, 40%, 45%, 50%, 60%, 70%, 80%, 90%, 95%, or 99% increase in ocular function in a subject.
f) A reduction or slowing of the rate of disease progression in a subject with an ocular disease.
g) A reduction, slowing or prevention of ocular dysfunction. In aspects of the invention, the cyclohexanehexol compound or medicament induces at least about a 2%, 5%, 10%, 15%, 20%, 30%, 40%, 50%, 60%, 70%, 80%, or 90% reduction or slowing of ocular dysfunction.
h) A reduction or inhibition of VEGF or VEGF activity. In aspects of the invention, the cyclohexanehexol compound or medicament induces at least about a 1%, 1.5%, 2%, 5%, 10%, 15%, 20%, 30%, 40%, 50%, 60%, 70%, 80%, or 90% reduction in VEGF or VEGF activity.
i) An increase in survival or longevity in a subject with symptoms of an ocular disease.

In aspects of the invention beneficial effects of a medicament or treatment of the invention can manifest as (a) and (b); (a), (b) and (c); (a), (b), (c) and (d); (a), (b), (c), (d), (e) and (f); (a), (b), (c), (d), (e), (f) and (g); (a) to (h), or (a) to (i).

Cyclohexanehexol compounds, medicaments and methods of the invention can be selected that have sustained beneficial effects, preferably statistically significant sustained beneficial effects. In an embodiment, a medicament is provided comprising a therapeutically effective amount of a cyclohexanehexol compound that provides a statistically significant sustained beneficial effect.

Greater efficacy and potency of a treatment of the invention in some aspects may improve the therapeutic ratio of treatment, reducing untoward side effects and toxicity. Selected methods of the invention may also improve long-standing ocular disease even when treatment is begun long after the appearance of symptoms. Prolonged efficacious treatment can be achieved in accordance with the invention following administration of a cyclohexanehexol compound or medicament comprising same.

In an aspect, the invention relates to a method for treating an ocular disease comprising contacting amyloid oligomers or aggregates in the retina, in particular macula, in a subject with a therapeutically effective amount of a cyclohexanehexol compound or a medicament of the invention.

In another aspect, the invention provides a method for treating an ocular disease by providing a medicament comprising a cyclohexanehexol compound in an amount sufficient to disrupt amyloid oligomers and/or aggregates for a prolonged period following administration.

In a further aspect, the invention provides a method for treating an ocular disease in a patient in need thereof which includes administering to the individual a medicament that provides a cyclohexanehexol compound in a dose sufficient to increase ocular function. In another aspect, the invention provides a method for treating an ocular disease comprising administering, preferably intraocularly, an amount of a cyclohexanehexol compound to a mammal, to reduce accumulation of amyloid and/or amyloid oligmers and/or aggregates in ocular cells for a prolonged period following administration.

The invention in an embodiment provides a method for treating an ocular disease, the method comprising administering to a mammal in need thereof a medicament comprising a cyclohexanehexol compound in an amount sufficient to reduce ocular dysfunction for a prolonged period following administration, thereby treating the ocular disease.

In another aspect, the invention provides a method for preventing and/or treating an ocular disease, the method comprising administering to a mammal in need thereof a medicament comprising a cyclohexanehexol compound in an amount sufficient to disrupt oligomerized and/or aggregated amyloid in ocular cells for a prolonged period following administration; and determining the amount of oligomerized and/or aggregated amyloid, thereby treating the ocular disease. The amount of oligomerized and/or aggregated amyloid may be measured using an antibody specific for amyloid or a cyclohexanehexol compound labeled with a detectable substance.

A method is provided for treating a subject with an ocular disease, comprising administering to the subject a therapeutically effective amount of a cyclohexanehexol compound, wherein the subject has failed to respond to previous treatment with conventional therapeutic agents or procedures, thereby treating the subject. In an aspect, a method is provided for treating a subject with age-related macular degeneration, comprising administering to the subject a therapeutically effective amount of a cyclohexanehexol compound, wherein the subject has failed to respond to previous treatment with conventional therapeutic agents or procedures, thereby treating the subject.

A method of treating an ocular disease in a subject in need thereof comprising intraocularly injecting a composition consisting essentially of a cyclohexanehexol compound in a pharmaceutically acceptable formulation and in an amount effective to treat an ocular disease without substantial toxicity to the patient. In an aspect, a method is provided for treating a subject with age-related macular degeneration (AMD), comprising intraocularly injecting a composition consisting essentially of a cyclohexanehexol compound in a pharmaceutically acceptable formulation and in an amount effective to treat AMD without substantial toxicity to the patient.

The present invention also includes methods of using the medicaments of the invention in combination with one or more additional therapeutic agents, in particular conventional therapeutic agents or procedures. In aspects of the invention for treating glaucoma, a subject may also receive conventional surgery or laser procedures. In other aspects of the invention, a subject is treated using a pharmacological approach. Examples of this approach for treating glaucoma include administration of cholinergic agents (e.g., pilocarpine), oral carbonic anhydrase inhibitors (e.g., acetazolamide (Diamox), dorzolamide (Trusopt), brinzolamide (Azopt)), topical beta-adrenergic receptor antagonists (e.g., timolol, levobunolol (Betagan), and betaxolol), alpha-2 adrenergic agonists (e.g., apraclonidine and brimonidine), cyclosporine A (cyclosporine, topical formulation Arrestase) and prostaglandin agonists (e.g., latanoprost (Xalatan), bimatoprost (Lumigan) and travoprost (Travatan)). [Examples of pharmacological approaches are disclosed in Khaw et al., BMJ320, 1619 (2000) and Khaw et al., BMJ 328, 156 (2004)].

In aspects of the invention for treating macular degeneration, a subject may also receive photocoagulation therapy or photodynamic therapy (see for example, US Patent Nos. 5,756,541, 5,910,510, 6,599,891, 7,060,695, 7,015,240, US Published Applications Nos. 20030087889 and 20040019032). For example, a subject may receive photodynamic therapy that uses verteporfin as the photosensitizer (e.g. Visudyne Photodynamic Therapy (Novartis)). A patient may receive macular translocation surgery or may be treated using rheophoresis. Carotenoids, such as lutein and zeaxanthin, which are potent antioxidants found in high concentrations in the macular retina may also be administered to a subject [See, for example, Chopdar et al., BMJ 326, 485 (2003)]. A subject may also receive anti-vascular endothelial growth factor (anti-VEGF) therapeutics in combination with a cyclohexanehexol compound. Examples of anti-VEGF therapeutics include pegaptanib (Macugen), ranibizumab (Lucentis), bevacizumab (Avastin). In some aspects, Triamcinolone (Kenalog) may be administered in combination with a cyclohexanehexol compound.

A method is provided for prolonging in a subject efficacy of a conventional therapy for treating an ocular disease (e.g. AMD) comprising administering to the subject receiving the conventional therapy a therapeutically effective amount of a cyclohexanehexol compound, preferably a therapeutically effective amount to prolong the efficacy of the conventional therapy or increase time to relapse. In an aspect, the subject suffers from AMD. In a particular aspect the subject is receiving an anti-VEGF therapeutic, in particular Lucentis. The therapy and cyclohexanehexol compound may be administered simultaneously or sequentially, in any order and for any period of time. In an aspect, the cyclohexanehexol compound is administered (e.g. for a period of time or continuously) following completion of the conventional therapy.

The invention also contemplates the use of a medicament comprising at least one cyclohexanehexol compound for treating an ocular disease or for the preparation of a medicament in treating an ocular disease. In an embodiment, the invention relates to the use of a therapeutically effective amount of at least one cyclohexanehexol compound for providing therapeutic effects, in particular beneficial effects, in treating an ocular disease or for the preparation of a medicament for providing therapeutic effects, in particular beneficial effects, in treating an ocular disease. In a still further embodiment the invention provides the use of a cyclohexanehexol compound for prolonged or sustained treatment of an ocular disease or for the preparation of a medicament for prolonged or sustained treatment of an ocular disease.

Therapeutic efficacy and toxicity of medicaments and methods of the invention may be determined by standard pharmaceutical procedures in cell cultures or with experimental animals such as by calculating a statistical parameter such as the ED₅₀ (the dose that is therapeutically effective in 50% of the population) or LD₅₀ (the dose lethal to 50% of the population) statistics. The therapeutic index is the dose ratio of therapeutic to toxic effects and it can be expressed as the ED₅₀/LD₅₀ ratio. Medicaments which exhibit large therapeutic indices are preferred.

### Administration

Cyclohexanehexol compounds and medicaments of the present invention can be administered by any means that produce contact of the active agent(s) with the agent's sites of action in the body of a subject or patient to produce a therapeutic effect, in particular a beneficial effect, in particular a sustained beneficial effect. Methods of administration include without limitation, systemic, transpleural, intravenous, oral, intraarterial, intramuscular, topical, via inhalation (e.g., as mists or sprays), via nasal mucosa, subcutaneous, transdermal, intraperitoneal, gastrointestinal, and directly to the eye or tissues surrounding the eye. The cyclohexanehexol compounds may be administered in the form of tablets, pills, powders, capsules, granules, injectables, creams, solutions, suppositories, emulsions, dispersions, food premixes, and in other suitable forms. The compounds can be administered in liposome formulations. The cyclohexanehexol compounds can also be administered as prodrugs.

In aspects of the invention, cyclohexanehexol compounds or medicaments are administered to the eye or tissues associated with the eye. The compounds and medicaments may be administered topically to the eye and may be in the form of eye drops or eye washes. The compounds and medicaments may also be administered by injection to the eye (intraocular injection) or to the tissues associated with the eye. They may also be administered by subconjunctival injection, trans-septal injection, intravitreal injection, transpleural injection, subretinal injection, periocular injection, sub-Tenon's injection, or retrobulbar injection. The cyclohexanehexol compounds and medicaments may also be administered to a subject as an implant which is preferably a biocompatible and/or biodegradable sustained release formulation which gradually releases the compounds over a dosage period. Implants for ocular administration are well-known in the art; see for example, US Patent Nos. 5,501,856, 5476,511 and 6,331,313. Cyclohexanehexol compounds may also be administered using iontophoresis, for example using the methods described in US Patent No. 4,454,151, and US Patent Application Publication Nos. 20030181531 and 20040058313.

A cyclohexanehexol compound and medicament of the invention can be formulated for sustained release, for delivery locally or systemically. It lies within the capability of a skilled physician or veterinarian to select a form and route of administration that optimizes the effects of the medicaments and treatments to provide therapeutic effects, in particular beneficial effects, more particularly sustained beneficial effects.

The dosage regimen of the invention will vary depending upon known factors such as the pharmacodynamic characteristics of the selected cyclohexanehexol compounds and their mode and route of administration; the species, age, sex, health, medical condition, and weight of the patient, the nature and extent of the symptoms, the kind of concurrent treatment, the frequency of treatment, the route of administration, the renal and hepatic function of the patient, and the desired effect.

An amount of a cyclohexanehexol compound which will be effective in the treatment of an ocular disease to provide effects, in particular beneficial effects, more particularly sustained beneficial effects, can be determined by standard clinical techniques. The precise dose to be employed in the formulation will also depend on the route of administration, and the seriousness of the disease, and will be decided according to the judgment of the practitioner and each patient's circumstances.

Suitable dosage ranges for administration are particularly selected to provide therapeutic effects, in particular beneficial effects, more particularly sustained beneficial effects. A pharmaceutical unit dosage of a cyclohexanehexol compound is preferably fabricated and administered to provide a defined final concentration of the drug either in the blood, or in tissues of the eye and/or tissues associated with the eye.

A dosage range is generally effective for triggering the desired biological responses. The dosage ranges may generally be about 0.001 µg to about 5 g per kg per day, about 0.01 µg to about 5 g per kg per day, about 0.1 µg to about 5 g per kg per day, about 0.1 mg to about 5 g per kg per day, about 0.1 mg to about 2 g per kg per day, about 0.5 mg to about 5 g per kg per day, about 1 mg to about 5 g per kg per day, about 1 mg to about 500 mg per kg per day, about 1 mg to about 200 mg per kg per day, about 1 mg to about 100 mg per kg per day, about 5 mg to about 100 mg per kg per day, about 10 mg to about 100 mg per kg, about 25 mg to about 75 mg per kg per day, about 1 mg to about 50 mg per kg per day, about 2 mg to about 50 mg/kg/day, about 2 mg to about 40 mg per kg per day, or about 3 mg to about 25 mg per kg per day. In aspects of the invention, the dosage ranges are generally about 0.01 µg to about 2 g per kg, about 1 µg to about 2 g per kg, about 1 mg to about 2 g per kg, 5 mg to about 2 g per kg, about 1 mg to about 1 g per kg, about 1 mg to about 200 mg per kg, about 1 mg to about 100 mg per kg, about 1 mg to about 50 mg per kg, about 10 mg to about 100 mg per kg, or about 25 mg to 75 mg per kg of the weight of a subject. A medicament or cyclohexanehexol compound may be administered once, twice or more daily, in particular once daily.

In some aspects of the invention, the dosage ranges of a compound disclosed herein, administered once twice, three times or more daily, especially once or twice daily, are about 0.01 µg to 5 g/kg, 1 µg to 2 g/kg, 1 to 5 g/kg, 1 to 3 g/kg, 1 to 2 g/kg, 1 to 1 g/kg, I to 600 mg/kg, I to 500 mg/kg, 1 to 400 mg/kg, 1 to 200 mg/kg, 1 to 100 mg/kg, 1 to 90 mg/kg, 1 to 80 mg/kg, 1 to 75 mg/kg, 1 to 70 mg/kg, I to 60 mg/kg, 1 to 50 mg/kg, 1 to 40 mg/kg, 1 to 35 mg/kg, 1 to 30 mg/kg, 3 to 30 mg/kg, 3 to 20 mg/kg, I to 20 mg/kg, or 1 to 15 mg/kg. In embodiments of the invention, the required dose of a compound disclosed herein administered twice daily is about 1 to 50 mg/kg, 1 to 40 mg/kg, 2.5 to 40 mg/kg, 3 to 40 mg/kg, or 3 to 30 mg/kg. In embodiments of the invention, the required daily dose of the compound is about 0.01µg to 5 g/kg, 1µg to 5 mg/kg, or 1 mg to 1g/kg and within that range 1 to 500 mg/kg, 1 to 250 mg/kg, 1 to 200 mg/kg, 1 to 150 mg/kg, I to 100 mg/Kg, 1 to 70 mg/kg, 1 to 65 mg/kg, 2 to 70 mg/kg, 3 to 70 mg/kg, 4 to 65 mg/kg, 5 to 65 mg/kg, or 6 to 60 mg/kg.

In some aspects of the invention, the dosage ranges of a cyclohexanehexol compound administered once twice, three times or more daily, especially once or twice daily, are about 1 to 100 mg/kg, 1 to 90 mg/kg, 1 to 80 mg/kg, 1 to 75 mg/kg, 1 to 70 mg/kg, I to 60 mg/kg, 1 to 50 mg/kg, 1 to 40 mg/kg, 1 to 35 mg/kg, 2 to 35 mg/kg, 2.5 to 30 mg/kg, 3 to 30 mg/kg, 3 to 20 mg/kg, or 3 to 15 mg/kg.

In embodiments of the invention, the dosage ranges for the cyclohexanehexol compound are about 0.1 mg to about 2 kg per kg per day, about 0.5 mg to about 2 g per kg per day, about 1 mg to about 1 g per kg per day, about 1 mg to about 200 mg per kg per day, about 1 mg to about 100 mg per kg per day, about 10 mg to about 100 mg per kg per day, about 30 mg to about 70 mg per kg per day, about 1 mg to about 50 mg per kg per day, about 2 mg to about 50 mg per kg per day, about 2 mg to about 40 mg per kg per day, or about 3 mg to 30 mg per kg per day.

In embodiments of the invention, the required dose of cyclohexanehexol compound administered twice daily is about 1 to about 50 mg/kg, 1 to about 40 mg/kg, 2.5 to about 40 mg/kg, 3 to about 40 mg/kg, 3 to about 35 mg/kg, in particular about 3 to about 30 mg/kg.

In other embodiments of the invention, the required daily dose of cyclohexanehexol compound, is about 1 to about 80 mg/kg and within that range 1 to about 70 mg/kg, 1 to about 65 mg/kg, 2 to about 70 mg/kg, 3 to about 70 mg/kg, 4 to about 65 mg/kg, 5 to about 65 mg/kg, or 6 to about 60 mg/kg.

A cyclohexanehexol compound can be provided once daily, twice daily, in a single dosage unit or multiple dosage units (i.e., tablets or capsules) having about 50 to about 10000 mg, 50 to about 2000 mg, 70 to about 7000 mg, 70 to about 6000 mg, 70 to about 5500 mg, 70 to about 5000 mg, 70 to about 4500 mg, 70 to about 4000 mg, 70 to about 3500 mg, 70 to about 3000 mg, 150 to about 2500 mg, 150 to about 2000 mg, 200 to about 2500,200 to about 2000 mg, 200 to about 1500 mg, 700 to about 1200 mg, or 1000 mg, in particular 200 to 2000 mg, more particularly 700 to 1200 mg, most particularly 1000 mg.

In aspects of the invention, dosages which can be used for systemic administration include, without limitation, an effective amount within the dosage range of about 0.1 µg/kg to about 300 mg/kg, or within about 1.0 µg/kg to about 40 mg/kg body weight, or within about 10 µg/kg to about 20 mg/kg body weight, or within about 0.1 mg/kg to about 20 mg/kg body weight, or within about 1 mg/kg to about 20 mg/kg body weight, or within about 0.1 mg/kg to about 10 mg/kg body weight, or within about within about 1 mg/kg to about 10 mg/hg body weight, or within about 0.1 µg/kg to about 10 mg/kg body weight.

In aspects of the invention, dosages which can be used for systemic administration when based on body surface area (expressed in square meters, or m²) include, but are not limited to, an effective amount within the dosage range of about 0.1 µg/m² to about 300 mg/m² body surface area, or within about 10 µg/m² to about 300 mg/m² body surface area, or within about 100 µg/m² to about 300 mg/m² body surface area, or within about 1 mg/m² to about 300 mg/m² body surface area, or within about 10 mg/m² to about 300 mg/m² body surface area, or within about 10 mg/m² to about 200 mg/m² body surface area, or within about 10 mg/m² to about 120 mg/m² body surface area, or within about 40 mg/m² to about 120 mg/m² body surface area, or within about 60 mg/m² to about 100 mg/m² body surface area.

In other aspects of the invention for intraocular and intravitreous administration or injection, examples of dosages which can be used include, without limitation, about any of 1 µg, 5 µg, 10 µg, 15 µg, 20 µg, 25 µg, 30 µg, 50 µg, 75 µg, 100 µg, 200 µg, 300 µg, 400 µg, 500 µg, 600 µg, 700 µg, 800 µg, 900 µg, 1 mg, 2 mg, 3 mg, 4 mg, or 5 mg per eye. For periocular administration or injection, examples of dosages which may be used include, without limitation, about any of 25 µg, 50 µg, 100 µg, 150 µg, 200 µg, 250 µg, 300 µg, 350 µg, 400 µ.g, 500 µg, 600 ug, 700 µg, 750 µg, 800 µg, 900 µg, 1 mg, 1.5 mg, 2 mg, 2.5 mg, 3 mg, 3.5 mg, 4 mg, 4.5 mg, 5 mg, 6 mg, 7 mg, 8 mg, 9 mg, 10 mg, 12.5 mg, 15 mg, 20 mg, 25 mg, 30 mg, 40 mg, or 50 mg per eye.

A medicament or treatment of the invention may comprise a unit dosage of at least one compound of the invention to provide beneficial effects. A "unit dosage" or "dosage unit" refers to a unitary i.e. a single dose, which is capable of being administered to a patient, and which may be readily handled and packed, remaining as a physically and chemically stable unit dose comprising either the active agents as such or a mixture with one or more solid or liquid pharmaceutical excipients, carriers, or vehicles.

A subject may be treated with a cyclohexanehexol compound or medicament thereof on substantially any desired schedule. A cyclohexanehexol compound or medicament of the invention may be administered one or more times per day, in particular 1 or 2 times per day, once per week, once a month or continuously. However, a subject may be treated less frequently, such as every other day or once a week, or more frequently. A cyclohexanehexol compound or medicament may be administered to a subject for about or at least about 1 week, 2 weeks to 4 weeks, 2 weeks to 6 weeks, 2 weeks to 8 weeks, 2 weeks to 10 weeks, 2 weeks to 12 weeks, 2 weeks to 14 weeks, 2 weeks to 16 weeks, 2 weeks to 6 months, 2 weeks to 12 months, 2 weeks to 18 months, 2 weeks to 24 months, or for more than 24 months, periodically or continuously.

In an aspect, dosages of cyclohexanehexol compounds may be administered in a sustained release formulation or a sustained release implant including an implant which gradually releases the compounds over a period of time and which allow the compounds to be administered less frequently, for example once a month, about once every 2-6 months, about once every year, or even a single administration which need not be repeated. Sustained release implants, devices or formulations may be administered by topical application to the eye by injection, or can be surgically implanted in various locations in the eye or tissues associated with the eye, such as intraocular, intravitreal, vitreous chamber, vitreous body, subretinal, periocular, retrobulbar, subconjunctival or subTenons. A sustained release formulation may be combined with iontophoretic methods.

In an aspect, the invention provides a regimen for supplementing a human's diet, comprising administering to the human a supplement comprising a cyclohexanehexol compound or a nutraceutically acceptable derivative thereof. A subject may be treated with a supplement at least about every day, or less frequently, such as every other day or once a week. A supplement of the invention may be taken daily but consumption at lower frequency, such as several times per week or even isolated doses, may be beneficial. In a particular aspect, the invention provides a regimen for supplementing a human's diet, comprising administering to the human about 1 to about 1000, 5 to about 200 or about 25 to about 200 milligrams of a cyclohexanehexol compound, or nutraceutically acceptable derivative thereof on a daily basis. In another aspect, about 50 to 100 milligrams of a cyclohexanehexol compound is administered to the human on a daily basis.

A supplement of the present invention may be ingested with or after a meal. Thus, a supplement may be taken at the time of a person's morning meal, and/or at the time of a person's noontime meal. A portion may be administered shortly before, during, or shortly after the meal. For daily consumption, a portion of the supplement may be consumed shortly before, during, or shortly after the human's morning meal, and a second portion of the supplement may be consumed shortly before, during, or shortly after the human's noontime meal. The morning portion and the noontime portion can each provide approximately the same quantity of a cyclohexanehexol compound. A supplement and regimens described herein may be most effective when combined with a balanced diet according to generally accepted nutritional guidelines, and a program of modest to moderate exercise several times a week.

In a particular aspect, a regimen for supplementing a human's diet is provided comprising administering to the human a supplement comprising, per gram of supplement: about 5 milligram to about 50 milligrams of one or more cyclohexanehexol compound or a nutraceutically acceptable derivative thereof. In an embodiment, a portion of the supplement is administered at the time of the human's morning meal, and a second portion of the supplement is administered at the time of the human's noontime meal.

The invention will be described in greater detail by way of specific examples. The following examples are offered for illustrative purposes, and are not intended to limit the invention in any manner.

### EXAMPLES

### Example 1

### Eye Drops

Solution compositions for topical administration containing AZD103 or ELND005 can be prepared as illustrated below:

| | | |
|---|---|---|
| AZD 103/ ELND005 | 6400 | mg |
| 0.5% hydroxyethylcellulose | 1 | L |

AZD103/ELND005 may be dissolved directly into 0.5% hydroxyethylcellulose to form a solution. The formulation can be rendered sterile by using sterile components and proceeding under sterile conditions.

Additional eyedrop formulations may be prepared having the following composition:

| | |
|---|---|
| AZD103/ ELND005 | 0.5% |
| Benzalkonium chloride solution | 0.02% v/v |
| Disodium edentate | 0.05% |
| NaCl | 0.8% |
| Water | to 100% |

### Example 2

### Effect of AZD103/ELND005 on toxicity of nonfibrillar amyloid oligomers to human primary retinal pigmented epithelium.

The effect of AZD103/ELND005 on the toxicity of amyloid oligomers in cultured SH-SY5Y human neuroblastoma cells and human primary RPE cells will be assessed spectrophotometrically using a 3-[4,5-dimethylthiazol-2-yl]-2,5-diphenyl tetrazolium bromide-based (MTT-based) assay (Sigma Aldrich; see also Luibl , V. et al, J Clin Invest., 2006, 116: 378). RPE cells may be obtained from Advanced Bioscience Resources Inc. and they may be maintained in DMEM supplemented with 2 mM L-glutamine, 100 U/ml penicillin, 100µg streptomycin, and 10% fetal bovine serum at 37°C.

### Example 3

### Topical admunistration of AZD103 or ELND005 for suppressing choroidal neovascularization and retinal leaks.

Formulations of AZD103 or ELND005 will be tested in a model of choroidal angiogenesis in which angiogenesis is induced using laser-induced rupture of the Bruch's membrane of C57BL/6 mice. In particular, 4 to 5 week old female C57BL/6J mice (n=10/group) will be delivered three bums of 532 nm diode laser photocoagulation at the 9, 12, and 3 o'clock positions of the posterior pole of the retina. After laser burn, mice will be treated with vehicle or an AZD103 or ELND005 formulation. After 2 weeks, mice will be perfused with fluorescein-labeled dextran, and choroidal flatmounts will be analyzed using image analysis software to recognize fluorescently stained neovascularization and the total area of neovascularization per retina will be calculated.

### Example 4

The pathology of glaucoma involves the loss of the retinal ganglion cells (RGCs). Glaucoma is commonly linked to elevated intraocular pressure, which may lead to loss of RGCs through local accumulation of amyloid. The potential of AZD-103 will be determined by examining its effects on RGC apoptosis induced in two ways: direct administration of amyloid into the eye and experimental elevation of intraocular pressure.

Amyloid administration: Freshly-made Aβ1-42 oligomers (0.55nM) will be injected intravitreally into Dark Agouti rats. Contralateral eyes will be used as controls, injected with water rather than Aβ. Animals will be assessed at various timepoints up to 72 hours after injection of amyloid, and the level of RGC apoptosis quantified. Apoptosing retinal cells will be labeled by intravitreal injection of Alexa Fluor 488-labelled annex in 5 and detected in real time using a confocal laser scanning ophthalmoscope. A small number of animals will be sacrificed after each observation timepoint for confirmation of RGC apoptosis by conventional histological techniques. The number of apoptotic RGCs will be counted manually by blinded observers. The amount of RGC apoptosis will be expressed in terms of density or percentage of total RGC count. To determine the effect of AZD-103 in this system, rats will be treated with AZD-103 in drinking water at 10 mg/ml, ad libitum, for 7 days prior to the amyloid administration, and then through to completion of the observations.

Experimental elevation of intraocular pressure: Unilateral elevation of intraocular pressure will be induced by injection of hypertonic saline (1.8M) into episcleral veins. Contralateral eyes will serve as controls. Intraocular pressure will be monitored regularly with aTonopen XL to permit determination of integral intraocular pressure. Animals will be assessed for RGC apoptosis, as above, at 2, 4, 8, 12 and 24 weeks. To determine the effect of AZD-103 in this system, rats will be treated with AZD-103 in drinking water at 10 mg/ml, ad libitum, for 7 days prior to injection of saline, and then through to completion of the observations.

### Example 5

Symptoms of age-related macular degeneration result from the loss of photoreceptors in the retina. The loss or dysfunction of the retinal pigment epithelial (RPE) layer removes vital trophic support for the photoreceptors, triggering their decline, and causing progressive vision loss (dry AMD). Alternatively, VEGF expressed by the RPE induces neovascularization into the retina. The leakage of fluid from these vessels leads to sudden loss of vision (wet AMD). In both pathologies, the RPE appears to be a key cell layer. As a further commonality between the disease types, the pathological hallmark of all AMD is the deposition of drusen: extracellular deposits adjacent to (outside of) the RPE layer. Amyloidβ is one of the key constituents of drusen and distinguishes between the deposits observed in AMD from those occasionally observed in normal eyes. Aβ is known to be toxic to a number of cell types, including primary RPE cells, and has also been shown to induce expression of VEGF from RPE cells. Aβ may therefore play a role in the pathology of both dry and wet AMD, through its effects on RPE cells.

The potential of AZD-1 03 to neutralize the effects of Aβ on VEGF expression by RPE cells was investigated using the following materials and methods.
*Materials: Human VEGF Duoset, 2^{nd} Generation* was obtained from R & D Systems [#DY293B]. 3,3',5,5'- Tetramethylbenzidine Liquid Substrate (TMB) was obtained from Sigma [#S8865]. The stop solution forTMB was also obtained from Sigma [#S5814]. *Tween-*20 was obtained from Fisher [#BP 337-500]; *Probumin* was obtained from Millipore [#82-045-1]; and, *10X Phosphate Buffered Saline(PBS)* was obtained from Roche [# 11666789001].
*Cell Culture:* A spontaneously arising retinal pigment epithelia cell line, ARPE-19 which expresses RPE-specific markers; CRALBP and RPE-65(ATCC CRL-2302), was maintained in a 1:1 Mixture of Dulbecco's modified Eagles medium and Ham's F12 medium with HEPES buffer containing 10% Fetal Bovine Serum, 100U/ml penicillin, and 100ug/ml streptomycin in 5% CO₂ at 37°C.
*Amyloid beta 1-42 oligomers:* 1 mg of synthetic amyloid beta 1-42 (AnaSpec, San Jose, CA) was dissolved in 1 ml of serum-free DMEM/F 12, vortexed and sonicated for 10 minutes. The mixture was then incubated overnight at 37°C with gentle shaking. Medium after incubation was centrifuged at 14000rpm for 10min to remove any insoluble aggregates. Oligomer solution was used the same day for the assay.
*Cell Treatments:* ARPE-19 cells (passage 12 to 14) were sub-cultured into 96-well tissue culture plates at a density of 1.5 x 10⁴ cells. Cells were incubated over-night at 37°C in a 5%CO₂ incubator, in order to allow cells to adhere. The following day, all media was changed and cells were incubated in serum-free DMEM/F12 in the presence or absence of Aβ₁₋₄₂ oligomers at concentrations ranging from 1µg/m) to 20µg/ml. There were also cells that were treated with A_{β1-42} that had been pre-incubated for 2 hours at 37°C with AZD 103 at treatment ratios (weight to weight ratios) of *1:0.5, 1: 1,* and *1:2.* Treated cells were incubated for an additional 24Hr at 37°C, 5%CO₂. Medium was then collected and centrifuged at 1000rpm for 10min to remove any cellular debris. The Media was then assessed for levels of VEGF by ELISA (R&D Systems, Minneapolis, MN)
*VEGF ELISA:* Conditioned media collected from cell cultures grown in the presence or absence of Aβ₁₋₄₂ and AZD103 were tested for vascular endothelial growth factor (VEGF) expression using a human VEGF duoset ELISA development kit as described by the manufacturer (R &D systems). Mouse anti-human VEGF was used as the capture antibody (1µg/ml) and biotinylated goat anti-human VEGF (100ng/ml) was used as the detection antibody. Supernatants were incubated with capture antibody (in 96 well) for 2 hours at room temperature. Wells were washed three times with wash buffer (0.05% Tween-20 in phosphate buffered saline (PBS) pH 7.4), followed by incubation with detection antibody for 2 hours at room temperature. Following three washes, the wells were incubated with Streptavidin-HRP for 20min. Subsequently, an addition of 3,3',5,5'- Tetramethylbenzidine Liquid Substrate (TMB) was made to each well and incubated for 30min at room temperature. The reaction was stopped and the colour development was read spectrophotometrically at a wavelength of 450nm.

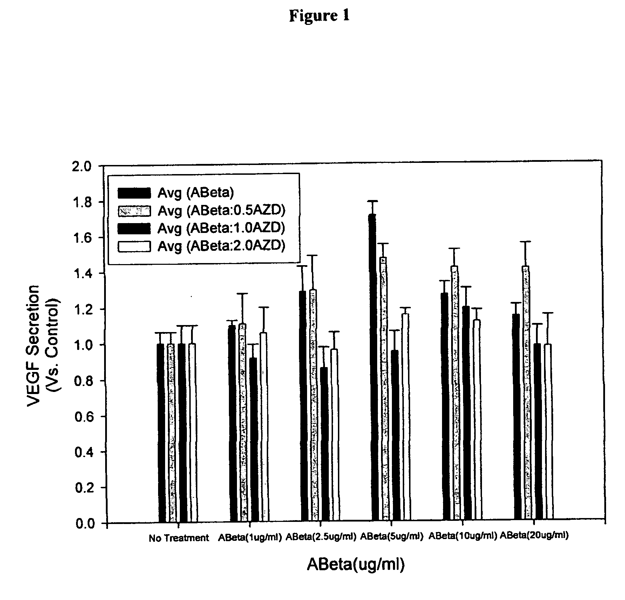

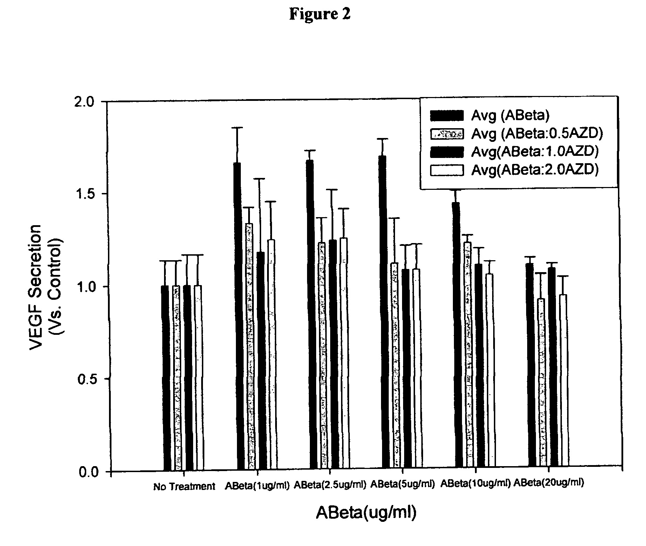

The results are illustrated in Figures 1 and 2. VEGF secretion is upregulated by increasing concentrations of freshly prepared Aβ₁₋₄₂ oligomers in serum-free media, up to a concentration of 5 ug/ml. Pre-incubation of AZD 103 with Aβ₁₋₄₂ is effective towards reducing the amount of secreted VEGF, without reducing secretions beyond the basal VEGF levels found in non-treated cells. VEGF secretions were analyzed as described and are expressed as a comparison to Normal ARPE-19 cells that received no treatment. AZD-103 appears to be able to neutralize the effects of Aβ on VEGF expression by RPE cells. This expression is returned to basal levels (i.e. comparable to expression levels in the absence of Aβ). These results indicate that AZD-103 may be able to prevent one of the molecular interactions that is pivotal in the initiation and continuation of wet AMD. More generally, AZD-103 can prevent Aβ from exerting potentially pathologic effects on RPE cells, and so may be of utility in both wet and dry AMD.

### Example 6

The ocular pathologies of AMD may be recapitulated in a murine model by applying three physiologically relevant risk factors: specific *APOE* genotype (APOE4), advanced age and high fat/cholesterol-rich (HF-C) diet. These mice develop sub-retinal pigment epithelium (RPE) deposits (basal deposits), RPE atrophy and choroidal neovascularization in a temporal, non-fully penetrant manner that is analogous to human AMD progression [Malek, G., et al., (2005), Proc Natl Acad Sci USA 102, 11900-5]. An electrophysiological phenotype is associated with this pathology. Electroretinogram (ERG) recordings *of APOE4* HF-C mice demonstrate statistically significant decreased a- and b-wave amplitudes [Ding JD et al, (2008), Vision Res. 48(3):339-45]. The ability of AZD-103 to prevent retina/RPE damage, the buildup of basal deposits and attenuation of the ERG will be evaluated.

Aged male *APOE4* mice housed conventionally, under ambient conditions maintained on water *ad libitum* and normal mouse chow (ND), will be assigned to three treatment groups. This assignment will be random, although the ages of the animals will be balanced across the groups. One group (n=9) will be maintained on the normal diet. The second group (n=12) will be switched to a HF-C diet (35% fat, 20% protein, 45% carbohydrates, 1.25% cholesterol, 0.5% sodium cholate) for 8 weeks. The third group (n=15) will receive AZD-103 ad libitum (dissolved in drinking water at 10 mg/ml) for 7 days. They will then be switched to the HF-C diet for 8 weeks, during which time they will continue to receive AZD-103 ad libitum. Animals will undergo assessments prior to dietary assignment, and after 8 weeks on the assigned diet. After this time all animals will be sacrificed. All animals will undergo the following assessments:
1. Fundus examination and photography before and after the assigned diet.
2. Total plasma cholesterol levels in whole blood of fasted animals before and after the assigned diet.
3. Full-field ERGs before and after the assigned diet. Animals will be dark adapted for at least 12 hours. Each animal will be anesthetized with a ketamine/xylazine cocktail, pupils dilated and the animal stabilized on a 37°C warming pad. ERG tracings will be recorded using a platinum iridium wire loop electrode placed in contact with the eye along with a drop of 2.5% hydroxypropyl methylcellulose. Mice will be placed in a photopic stimulator chamber where the animal is exposed to flashes of light (max intensity of 1000 cd-s/m2 attenuate in I log steps, starting from 0.0005). The a-wave amplitude is measured from baseline to the a-wave trough, and the b-wave amplitude is measured from the a-wave trough to the b-wave peak.
4. Postmortem immunohistochemical localization of proteins, including amyloid beta, other proteins associated with AMD lesions (vitronectin, apoE, apoB), and proteins associated with photoreceptor synaptic terminals: SV2 VGLUT1, PKCα.
5. Quantitation of photoreceptors. Eyes will be fixed and embedded in Epon-Spurr resin, cut at 500 nm and mounted on glass slides. Cross sections that bisect the optic nerve will be used for measurement of retinal layers and to count cell numbers. The thickness of outer nuclear layer and the linear density of photoreceptor will be cal cul ated.

Personnel responsible for ERG's and assessment of pathology will remain masked to the identity of treatment groups until they have finished data collection and assessment of disease severity.

The present invention is not to be limited in scope by the specific embodiments described herein, since such embodiments are intended as but single illustrations of one aspect of the invention and any functionally equivalent embodiments are within the scope of this invention. Indeed, various modifications of the invention in addition to those shown and described herein will become apparent to those skilled in the art from the foregoing description and accompanying drawings. Such modifications are intended to fall within the scope of the appended claims.

## Claims

1. Scyllo-inositol for use in treating macular degeneration.

2. Scyllo-inositol according to claim 1, wherein macular degeneration is age-related macular degeneration.

3. Scyllo-inositol according to claim 1, wherein age-related macular degeneration is wet age-related macular degeneration.

4. Scyllo-inositol according to claim 1, wherein age-related macular degeneration is dry age-related macular degeneration.

5. Scylloinositol according to any preceding claim which reduces vascular endothelial growth factor (VEGF) expression.

6. Scylloinositol according to claim 5, which returns VEFG expression to a basal level.

7. A medicament for use in treating macular degeneration comprising scyllo-inositol according to any preceding claim and an ophthalmologically acceptable carrier, excipient or vehicle.

8. A medicament according to claim 7 which is a topical ophthalmic formulation.

## Patentansprüche

1. scyllo-Inositol zur Verwendung bei der Behandlung von Makuladegeneration.

2. scyllo-Inositol nach Anspruch 1, wobei es sich bei der Makuladegeneration um altersbedingte Makuladegeneration handelt.

3. scyllo-Inositol nach Anspruch 1, wobei es sich bei der altersbedingten Makuladegeneration um feuchte altersbedingte Makuladegeneration handelt.

4. scyllo-Inositol nach Anspruch 1, wobei es sich bei der altersbedingten Makuladegeneration um trockene altersbedingte Makuladegeneration handelt.

5. scyllo-Inositol nach einem der vorhergehenden Ansprüche, das die Expression von VEGF (vascular endothelial growth factor) verringert.

6. scyllo-Inositol nach Anspruch 5, das die Expression von VEGF wieder auf ein Basalniveau zurückbringt.

7. Medikament zur Verwendung bei der Behandlung von Makuladegeneration, umfassend scyllo-Inositol nach einem der vorhergehenden Ansprüche und einen ophthalmologisch unbedenklichen Träger, einen ophthalmologisch unbedenklichen Hilfsstoff oder ein ophthalmologisch unbedenkliches Vehikel.

8. Medikament nach Anspruch 7, bei dem es sich um eine topische ophthalmische Formulierung handelt.

## Revendications

1. Scyllo-inositol pour utilisation dans le traitement de la dégénérescence maculaire.

2. Scyllo-inositol selon la revendication 1, la dégénérescence maculaire étant la dégénérescence maculaire liée à l'âge.

3. Scyllo-inositol selon la revendication 1, la dégénérescence maculaire liée à l'âge étant la dégénérescence maculaire liée à l'âge humide.

4. Scyllo-inositol selon la revendication 1, la dégénérescence maculaire liée à l'âge étant la dégénérescence maculaire liée à l'âge sèche.

5. Scyllo-inositol selon l'une quelconque des revendications précédentes qui réduit l'expression du facteur de croissance endothélial vasculaire (VEGF).

6. Scyllo-inositol selon la revendication 5, qui ramène l'expression de VEGF à un niveau basal.

7. Médicament pour utilisation dans le traitement de la dégénérescence maculaire comprenant du scyllo-inositol selon l'une quelconque des revendications précédentes et un support, excipient ou véhicule acceptable en ophtalmologie.

8. Médicament selon la revendication 7 qui est une formulation ophtalmique topique.
